(19) Europäisches Patentamt
European Patent Office
Office européen des brevets

(11) **EP 3 824 082 B1**

## (12) EUROPEAN PATENT SPECIFICATION

(45) Date of publication and mention
of the grant of the patent:
**04.05.2022 Bulletin 2022/18**

(21) Application number: **18759188.8**

(22) Date of filing: **20.07.2018**

(51) International Patent Classification (IPC):
**C12N 11/00** $^{(2006.01)}$

(52) Cooperative Patent Classification (CPC):
**C12N 11/04; C12N 11/08; G01N 33/54313**

(86) International application number:
**PCT/PT2018/000017**

(87) International publication number:
**WO 2020/017985 (23.01.2020 Gazette 2020/04)**

(54) **COMBINED COMPOSITE FOR STABILIZATION OF ACTIVE BIOLOGICAL MATERIALS, METHOD OF PRODUCTION AND USE THEREOF**

KOMBINIERTES VERBUNDMATERIAL ZUR STABILISIERUNG VON AKTIVEN BIOLOGISCHEN MATERIALIEN, VERFAHREN ZU SEINER HERSTELLUNG UND SEINE VERWENDUNG

COMPOSITE COMBINÉ POUR LA STABILISATION DE MATÉRIAUX BIOLOGIQUES ACTIFS, SA MÉTHODE DE PRODUCTION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.05.2021 Bulletin 2021/21**

(73) Proprietors:
• **Alho de Andrade, Luís Filipe**
**1400-424 Lisboa (PT)**
• **GODINHO BARREIRA, Luis Gustavo**
**2840-430 Torre da Marinha (PT)**

(72) Inventor: **GODINHO BARREIRA, Luís Gustavo**
**2840-430 Torre Da Marinha (PT)**

(74) Representative: **Ferreira Magno, Fernando Antonio**
**A.G. da Cunha Ferreira, Lda.**
**Avenida José Gomes Ferreira 15, 3rd floor L**
**1495-139 Algés (PT)**

(56) References cited:
• **LI B ET AL: "NEW IMMOBILIZATION METHOD FOR ENZYME STABILIZATION INVOLVING A MESOPOROUS MATERIAL AND AN ORGANIC/INORGANIC HYBRID GEL", BIOTECHNOLOGY LETTERS, KLUWER ACADEMIC PUBLISHERS, DORDRECHT, vol. 22, no. 24, 1 January 2000 (2000-01-01), pages 1953-1958, XP008017673, ISSN: 0141-5492, DOI: 10.1023/A:1026702025928**
• **MUDITHA D. SENARATH-YAPA ET AL: "Dye leaching from a doped sol-gel is eliminated by conjugation to a dendrimer", ANALYTICA CHIMICA ACTA, vol. 432, no. 1, 1 March 2001 (2001-03-01), pages 89-94, XP055548394, AMSTERDAM, NL ISSN: 0003-2670, DOI: 10.1016/S0003-2670(00)01347-7**
• **JIN SHI ET AL: "A comparative study of enzyme immobilization strategies for multi-walled carbon nanotube glucose biosensors;A comparative study of enzyme immobilization strategies for multi-walled carbon nanotube glucose biosensors", NANOTECHNOLOGY, IOP, BRISTOL, GB, vol. 22, no. 35, 9 August 2011 (2011-08-09), page 355502, XP020210061, ISSN: 0957-4484, DOI: 10.1088/0957-4484/22/35/355502**

EP 3 824 082 B1

- GUPTA ET AL: "Entrapment of biomolecules in sol-gel matrix for applications in biosensors: Problems and future prospects", BIOSENSORS AND BIOELECTRONICS, ELSEVIER SCIENCE LTD. UK, AMSTERDAM, NL, vol. 22, no. 11, 30 March 2007 (2007-03-30), pages 2387-2399, XP022006432, ISSN: 0956-5663, DOI: 10.1016/J.BIOS.2006.12.025
- CHIEN LIANG-JUNG ET AL: "Biosilicification of dual-fusion enzyme immobilized on magnetic nanoparticle", BIOTECHNOLOGY AND BIOENGINEERING, WILEY, US, vol. 100, no. 2, 1 June 2008 (2008-06-01), pages 223-230, XP009120581, ISSN: 0006-3592, DOI: 10.1002/BIT.21750 [retrieved on 2007-12-13]
- TSAI-YIN LIN ET AL: "Entrapment of horseradish peroxidase in sugar-modified silica monoliths: Toward the development of a biocatalytic sensor", BIOSENSORS AND BIOELECTRONICS, vol. 22, no. 9-10, 1 April 2007 (2007-04-01), pages 1861-1867, XP055548398, AMSTERDAM, NL ISSN: 0956-5663, DOI: 10.1016/j.bios.2006.04.026

**Description**

## 1. Object of the invention

The present invention refers to the utilization of sol-gel synthesis to immobilize active or activable biological materials. More specifically the present invention refers to a new method, conceived to immobilize active or activable biological materials in a combined composite sol-gel in which the loss by leaching of the mentioned biological materials from that, combined composite is reduced while the biological activity of those materials is preserved.

## 2. State of the Art

Sol-gel matrices are highly porous materials mainly derived from silica whose synthesis conditions are recognized as benign for encapsulation of most biological molecules.

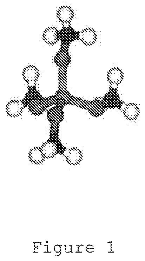

In a typical protocol of synthesis of sol-gel the precursors are alkoxides of the type $Si(OR)_4$ or alkoxysilanes of the type $XSi(OR)_3$ or $XX'Si(OR)_2$ in which X and X' refers to organic groups directly ligated to silica atom by a Si-C bridge in one top and presenting several other functional groups at the other top. Figure 1 illustrates one alkoxide where R-group is a methyl group: tetramethyl orthosilicate (TMOS).

The first reaction of sol-gel synthesis is hydrolysis of precursors [1] in which one OR ligand is replaced by OH group:

$$Si(OR)4 + H2O \leftrightarrow Si(OR)3(OH) + ROH$$

(*e.g.*)

$$Si(OCH_3)_4 + H_2O \leftrightarrow Si(OCH_3)_3(OH) + CH_3OH$$

Hydrolysis is then followed by condensation:

$$Si(OR)_3(OH) + Si(OR)_3(OH) \leftrightarrow (RO)_3SiOSi(OR)_3 + H_2O$$

(*e.g.*)

$$Si(OCH_3)_3(OH) + Si(OCH_3)_3(OH) \leftrightarrow (H_3CO)_3SiOSi(OCH_3)_3 + H_2O$$

Those hydrolysis and condensation are slow reactions but hydrolysis rate can be increased by an acid (e.g HCl) which donate positive charges capable of attacking oxygen of the alcoxyde group. Thus it is obtained a gel-of-silica with texture similar to a polymeric-gel. On the other hand protons acceptor media, an alkaline solution for instance, accelerates condensation rate leading to the formation of denser colloidal particles. The ability of controlling those kinetics is important to adapt encapsulation conditions for a better response of biological molecules.

Some additives have also demonstrated beneficial effects on biomolecules stability encapsulated in sol-gel: polyvinyl alcohol or glycerol can improve the activity of encapsulated lypases [2] [3]. Besides additives, the amount of water used to hydrolyse precursors can influence the activity of immobilized biomolecules and thus the available-water must assure the mobility of biomolecules in the matured matrices: with a low addition of water the activity of encapsulated $\beta$-galactosidase was higher at a recently gelified composite than at the respective matured composite [4].

One disadvantage associated to encapsulation techniques is the progressive loss of activity of the sol-gel matrix seen as an active-composite: the leach of encapsulated molecules result in decrease of activity of the matrix-composite and so limiting the efficiency on its long-standing use.

Several strategies have been come up to circumvent this hurdle in order to control the leaching of sol-gel encapsulated molecules. Some author aim to reduce leaching through decreasing pore-size by adjusting sol-gel composition and gelification/maturation conditions as referred by T.M. Butler et *al*. [5], Lobnik, I. Oehme et *al*. [6] and G.E. Badini et *al*. [7]. However the diffusion of analytes within matrices is reduced which results in longer response reaction times.

It was also proposed the covalent-link of biomolecules to a polycondensed net in the synthesis of sol-gel having somehow reduced the leach [8] [9] [10]. An alternative strategy was to increase the size of the immobilized biomolecules by linking to an inert macromolecular transporter [11] [12] [13] or yet an encapsulation within a supramolecular construct. [14]. However the activity of biomolecules requires some mobility and those approaches have reduced the expected activity.

Thus the problem this invention focused wets the stabilization of an active or activable biological material in a sol-gel matrix reducing so its loss by leaching and at the same time retaining its activity. That problem was solved by a new method of immobilization of active or activable biological materials in which a combined composite was obtained: one sol-gel immobilizing one other sol-gel previously produced within which active or activable biological materials were encapsulated in a good physical-chemical stability and response activity. B et al., Biotechnology Letters, 22(24):1953-1958 (2000) discloses horse radish peroxidase immobilised on mesoporous FSM-16 material which is then entrapped in DMDMOS/TMOS. Shi J et al., Nanotechnology, 22(35):355502 discloses combined nanotube network cast in TMOS composite structures entrapping glucose oxidase.

### 3. Description of the Invention

The therms "imobilization", "encapsulation", "imprisonment" and respective deductions here non-differentially used have the significance of stabilization of biological materials within a matrix of sol-gel composite.

With a double immobilization within sol-gel of an active or activable biological materials, reduction of its loss by leaching is expected. However it should also be expected a lesser access to the active sites of immobilized biological materials and consequently a decrease of its activity,

There were some practical difficulties on manufacturing such combined composite. By effecting a second immobilization in sol-gel of one other sol-gel, the simple composite, it complied a wide range of unsuccessful outcomes since the complete failure of gelification until inappropriate physical-chemical properties of the final materials.

Uncompromisingly to the theoretical basis, those non-successes would come from the highly higrophilic character of the simple composite which in the reaction conditions have induced a dislocation of water for its saturation resulting in a deficit of water required for hydrolysis of the orthosilicate precursor.

As result of an intensive research, the inventor verified that the method herein described produce a combined composite, one sol-gel immobilizing another sol-gel previously obtained encapsulating an active or activable biological materials with good physical-chemical stability and in which final formulation the loss of biological materials by leaching is reduced and respective activity is retained.

The present inventor surprisingly verified that double immobilization in a combined sol-gel composite of an active or activable biological materials obtained by the invented method not only remarkably reduced the loss by leaching of the mentioned materials but also preserved its activity in such a way that enabled at least 20 cycles of response from the immobilized biological material.

Thus according to the present invention it is provided a new method of production of a combined composite for stabilization of an active or activable biological material, which comprises the following steps:

a) to provide a simple composite under the shape of a sol-gel immobilizing an active or activable biological material of one or more different natures, finely grinded and at low available-water grade;

b) to provide a second sol-gel immobilizing the simple composite of a) including:

i) preparing a suspension of the simple composite of a) in phosphate buffer solution 100 mM pH = 6,8 $\pm$ 0,2 at concentration between 0,83% and 3,34% in reference to the volume of the final reacting mixture;

ii) effecting the hydrolysis of tetramethyl orthosilicate by acid catalysis adding HCl solution in a concentration of 4,50 mM to 8,34 mM in a proportion of 28% of the final volume of this mixture;

iii) adding the mixture ii) to equal volume of the suspension i) at 25°C to 30°C temperature allowing polymerization to occur until the combined composite obtained have consistency that enable to be fragmented;

iv) fragmenting the combined composite obtained in iii) at granulometry approximately equal or less than 2 mm$^3$ and approximately between 2 mm$^3$ and 4 mm$^3$.

v) optionally incubating the fragmented combined composite of iv) during 24 hours in a solution of bovine serum albumin (BSA) at 2,0% m/v solubilized in phosphate buffer saline 10 mM pH=7,3 in a proportion of three volumes of incubating solution to one volume of composite;

vi) maturating the combined composite by drying on a glass surface until a mass-decrease of circa 70% and available waiter grade of circa 20 ppm;

It is hence obtained a combined composite doubly immobilizing in a sol-gel matrix the active or activable biological material in which the loss by leaching of the mentioned material is reduced being additionally preserved its activity or activation capacity.

In a preferred embodiment of the invented method, the obtained combined composite doubly immobilizing in sol-gel an active or activable biological material, has a reduced loss of the mentioned material and preserved its activity for at least 20 cycles of utilization.

The active or activable biological materials that can be immobilized in the combined composite of the invention are not limited, allowing to be one or more of any immobilizable molecule in the simple composite, preferably one member of the specific ligation such as enzyme-substrate, antibody-antigen or any other pair of specific ligation preferably one biologically active protein, more preferably one enzyme or co-enzyme or one immunologically active protein of specific ligation, more preferably one antibody, one antigen or one hapten-protein.

The simple composite which will be immobilized in the combined composite is provided by any methodology known at the state-of-the-art that produces a hydrophilic composite preferably by acid hydrolysis of tetramethyl orthosilicate.

In a preferred embodiment of the invention the simple composite that will be immobilized in the combined composite and that by its turn it has immobilized active or activable biological materials, have a low grade of available-water between 10 and 15 ppm and is grinded to a granulometry between 100 and 110 $\mu m^3$.

The formation of the combined composite immobilizing the simple composite previously prepared is attained by acid hydrolysis of tetramethyl orthosilicate using HCl as catalyser in a concentration between 4,50 mM and 8,34 mM. That concentration is dictated by the water displacement in function of the protein immobilized in the simple composite.

When the biological material immobilized in the combined composite is one or more of the immunologically active proteins such as an antigen, an antibody, a hapten-protein, the method of the invention includes the additional step v) of incubation of the fragmented combined composite during 24 hours in bovine serum albumin at 2,0% m/v solubilized in phosphate buffer saline 10 mM pH=7,3 in a proportion of three volumes of the incubating solution to one volume of combined composite. This additional step elevates significantly the protein content of the matured combined composite until 3,0 to 3,5 relative to the protein content of the simple composite.

The present inventions refers as well to a combined composite doubly immobilizing an active or activable biological material in sol-gel obtained by the method of the invention. This combined composite is herein also referred as "doped" composite with the biological material which is immobilized within it.

The combined composite obtained by the method of this invention doubly immobilizing in sol-gel an active or activable biological material, presents a robustness and improved retention capacity of the referred active or activable immobilized biological material. At the same time the referred combined composite presents an internal structure, or porosity, that allows the permeation until the active sites of those immobilized biological materials, of an analyte (ligand, substrate or activating molecule specific of the active or activable biological material) present in an aqueous sample placed in contact with the referred combined composite, In other words the composite of this invention, presents not only an improved physical retention of the biological materials but also preserves the activity or the capacity of activation of those retained biological materials. The robustness of the combined composite of this invention together with its ability of maintaining the activity of the immobilized biological materials, enable the composite of this invention to be used along several successive utilization cycles.

In a preferred embodiment, one combined composite according to this invention can be used for at least 20 cycles of utilization.

It ought, to be intended as cycle of utilization of the doped composite each event of interaction/reaction among the biological material immobilized within the composite and the respective ligand or specific substrate, or other molecule of specific interaction (or analyte), by contacting the referred combined composite with a sample containing one analyte. For example, one cycle of utilization will be one cycle of an enzymatic reaction when the immobilized material is an enzyme or co-enzyme, one cycle of reaction of formation of an immune-complex when the immobilized biological material is an antibody, an antigen or a hapten-protein.

One cycle of utilization will preferably include steps of conditioning and washing of the combined composite before one next cycle. It might also include additional procedures for detection of the occurrence of the mentioned interaction/reaction.

The combined composite of the invention can hence be utilized in any application where will be utilized an active or activable biological material, more preferable when there will be utilized biomolecules in aqueous media, as for and without limited instance, the formation of immune-complexes in medical diagnosis, or the formation of products by biocatalysis with immobilized enzymes.

It has been widely recognized the vantages of biocatalysis relative to the conventional catalysis, that comes from the immobilization of enzymes and results in more catalytic efficiency, greater enzymatic stability, more selectivity of the substrates and minor costs of utilization by the less demanding thermal operational conditions and environmentally more friendly [15].

By this manner, in an embodiment of the combined composite of the invention it can be utilized as support of enzymes, thus with enzymatic activity along with several reaction cycles. Further it will be presented an example, but not limitative, with alkaline phosphatase.

In one of the preferred embodiment of the combined composite of this invention it will be utilized as support of immunologically active proteins, as for example antibodies, antigens or hapten-proteins, with activity in successive cycles of ligation to the respective ligands. So, in each cycle of utilization, it will be possible to detect the ligation of the specific ligands of those immunologically active proteins immobilized within the combined composite.

Being the, mentioned immunologically active proteins immobilized in the combined composite, or the respective ligands, associated to specific pathologies, the combined composite can be utilized in diagnosis of such diseases through the detection of the specific ligands present in a biological sample. In preference the biological sample will be from a human being or superior animal, more preferably a biological fluid such as plasma/serum from blood, urine, supernatant from tissue macerate or any other aqueous fluid without cells obtained from an individual for medical diagnosis proposes. The disease to be diagnosed can be any pathology that trigger the presence of antigens and/or antibodies in fluids and/or tissues of human being or superior animal.

So one utilization of this invented combined composite is at medical diagnosis which constitutes one preferential utilization.

In more detail but without limitation, the utilization of invented combined composite in medical diagnosis follows the principals of immune-diagnosis in that one biological sample to which it is suspected to contain the analytes of interest, for example antibodies, antigens or other analytes, is provided to get in contact with respective specific ligands. These last ones will be antigens, antibodies or any other specific ligands immobilized in the combined composite whose porosity enable the permeation of the mentioned analytes existent. in the aqueous liquid samples, until they contact with the referred specific immobilized ligands. Ones and others, or both, the analyte or the respective specific ligand will be associated to a pathology.

So by contact of the biological sample to be analysed with the combined composite doped with a specific ligand for a certain analyte, and that analyte being present in the aqueous sample it will be formed the specific complex analyte-ligand, for example an immune-complex antigen-antibody, that can further be detected and so elucidating the conclusion of the presence or the absence of the analyte in the biological sample and consequently the affirmative or negative, diagnostic of the associated disease.

The referred detection can be made by any adequate method such as colouring or fluorescence recurring for instance to labelled antibodies.

It will be further described an example of this embodiment in which the detection of the immune-complex (primary) formed after contact of a biological sample with the combined composite immobilizing an antigen (or antibody) is effected by a secondary labelled antibody (labelled with an enzyme e.g, peroxidase) that links specifically to the antibody (or antigen) of the biological sample consequently forming a secondary immune-complex. A further addition of a chromogenic substrate being degraded by the labelling enzyme of the secondary antibody provides a colourful appearance of the combined composite so that detection of the referred primary complex is accomplished.

The above mentioned detection can be validated by the absence of colour after applying the same biological sample at a same composite doped with a non-immunogenic protein.

Recycling of the combined composite enables it to be used in successive cycles of utilization. The combined composite of the invention is capable of being reused at least for 20 cycles of utilization as already described and as farther demonstrated.

In one aspect of this embodiment the combined composite of the invention doubly immobilizing one immunologically active protein, will be used as filling an analytical-chamber designed for detection of a specific ligand of the referred immunologically active protein (target-analyte) existent in a biological sample to which it will be put in contact along in such analytical-chamber,

The filling profile of the analytical-chamber with combined composite will be adjusted to the target-analyte and/or to the immobilized biological material, and will be determined by preliminary essays for adjusting operational rheology. In the same way, the protocol of use of the analytical-chamber, dilution-rates of reagents and biological samples will also be determined by routine preliminary essays for meeting criteria of Sensibility and Specificity, in function of the target-analyte and/or immobilized biological material.

Thus it will be determined the least content of simple composite to be immobilized in the combined composite that will enable to detect the existence of the analyte in the biological sample (Sensibility) and the greatest content of simple composite to be immobilized in the combined composite that will evidence no cross response between different analytes (Specificity) . At the same time it will be determined the most adequate profile of granulometry gradient that will fulfil the analytical-chamber.

So it is conceivable a device that will include a series of the above described analytical-chambers, preferably until 8 chamber disposed vertically in parallel and supplied on top by a common liquid-collector in which the liquid samples will be placed. Each analytical-chamber dedicated to detect one distinct analyte will be sided by a similar chamber dedicated to negative-control test which will be filled exactly with the same profile of granulometry gradient of combined composite immobilizing the same content of simple composite but doped with a non-immunogenic protein.

The device will hence be constituted by pairs of chambers, analytical and respective negative control chambers, being each analytical-chamber filled, with combined composite doubly immobilizing one different active or activable biological material as described above, and sided by a negative-control chamber filled with combined composite doubly immobilizing one non-immunologic responsive biological material. Such device will enable to detect a number preferably until 4, different target-analytes in one unique biological sample and in each cycle of utilization.

Thus one preferred embodiment of this invention is a portable and easily usable device, with no requirements of electric energy supply and that will enable to diagnose affirmatively or negatively in each biological sample, the existence of a number of analytes preferably until 4 as much as the number of analytical chambers that will comprise the construct. In particular, being each of the mentioned analytes related to pathologies, from one unique sample of biological fluid of a human being or superior animal to whom is suspected one specific pathology, this device will enable the differential diagnosis of until 4 pathologies. Additionally the device is reusable for at least 20 successive cycles of analytical utilization (diagnostic trials).

This device can be presented in a kit format which beyond, the device itself will include instructions of use and all the reagents required for the operation of diagnostic trials,

It will ahead be described some examples the preferred ways of putting in practice this invention and will also be reported several error-and-trial essays of the invention.

## 4. Description of the Figures

**Figure 1:** structural representation of an alkoxide, tetramethyl orthosilicate (TMOS).

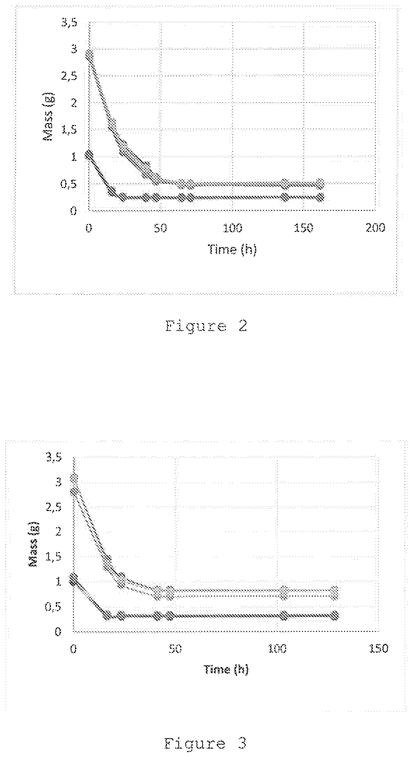

**Figure 2:** loss of mass at room exposition, maturation of the simple composite.

**Figure 3:** loss of mass at room exposition maturation of the combined composite.

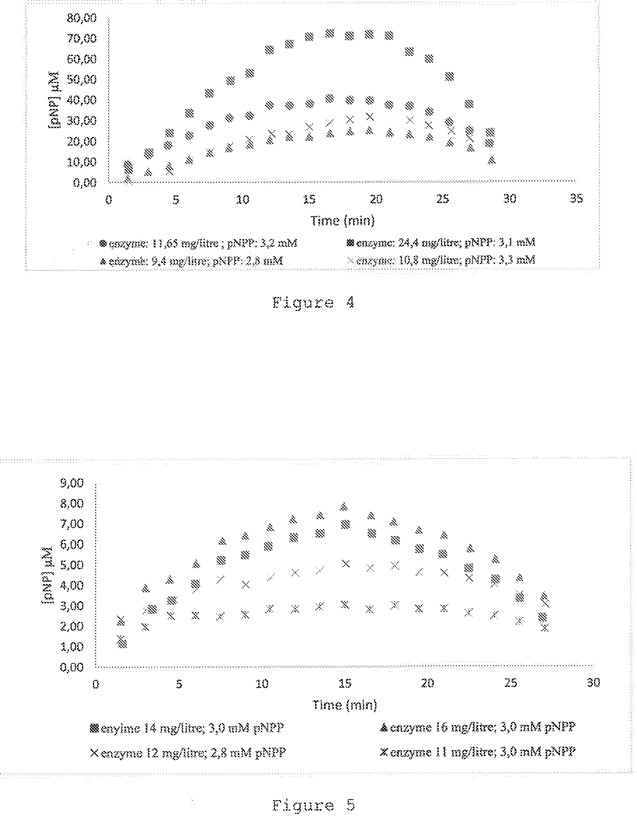

**Figure 4:** production of *p*-nitrophenol catalysed by alkaline phosphatase immobilized in simple composite (hydrolysis of *p*-nitrophenylphosphate in aqueous medium of phosphate buffer 100 mM pH=9,1) in relation to the mass of immobilized enzyme.

**Figure 5:** production of *p*-nitrophenol catalysed by alkaline phosphatase immobilized in combined composite (hydrolysis of *p*-nitrophenylphosphate in aqueous medium of phosphate buffer 100 mM pH=9,1) in relation to the mass of immobilized enzyme.

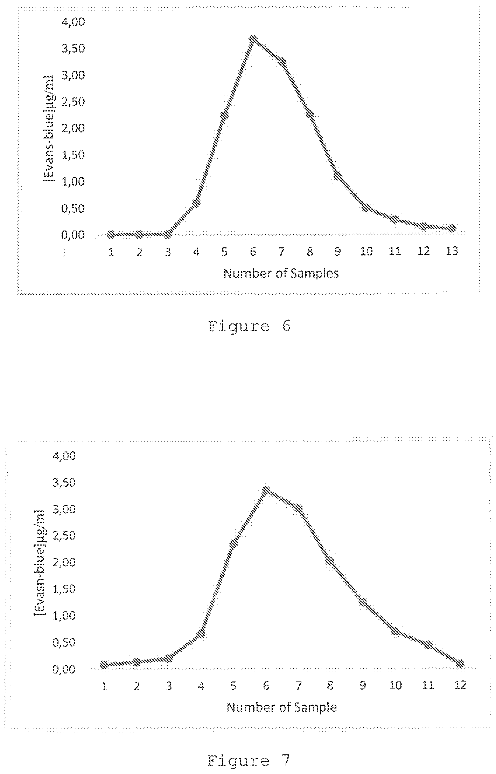

**Figure 6:** dye concentration of samples collected at 3 minutes intervals by the elution of 13 fractions of 1,0 ml of distilled water. The experience started with the elution of 1,0 ml of Evan-blue aqueous solution at concentration of 1,4 mg/100 ml by permeating a granulometry gradient of combined composite on ascendant layers (1,35 g of granulometry ($G$) > 1,0 mm$^3$; 1,80 g of 1,0 mm$^3$ > $G$ > 710 $\mu$m$^3$ ; central layer of 2,70g 710 $\mu$m$^3$ > $G$ > 212 $\mu$m$^3$ ; 1,60 g of 1,0 mm$^3$ > $G$ > 710 $\mu$m$^3$). The combined composite fulfilled a 12 cm$^3$ reactor of 4 cm$_{lengh}$ $\times$ 3 cm$_{width}$ $\times$ 1 cm$_{height:}$ dimensions.

**Figure 7:** dye concentration of samples collected at 30 seconds intervals by the elution of 12 fractions of 1,0 ml of distilled water. The experience started with the elution of 1,0 ml of Evan-blue aqueous solution at concentration of 1,4 mg/100 ml by permeating a granulometry gradient, of combined composite on ascendant, layers (1,35 g of granulometry ($G$) > 1,0 mm$^3$; 1,80 g of 1,0 mm$^3$ > $G$ > 710 $\mu$m$^3$ ; central layer of 2,70g 710 $\mu$m$^3$ > $G$ > 300 $\mu$m$^3$ ; 1,16 g of 1,0 mm$^3$ > $G$ > 710 $\mu$m$^3$) . The combined composite fulfilled a 12 cm$^3$ reactor of 4 cm$_{lengh}$ $\times$ 3 cm$_{width}$ $\times$ 1 cm$_{height}$ dimensions.

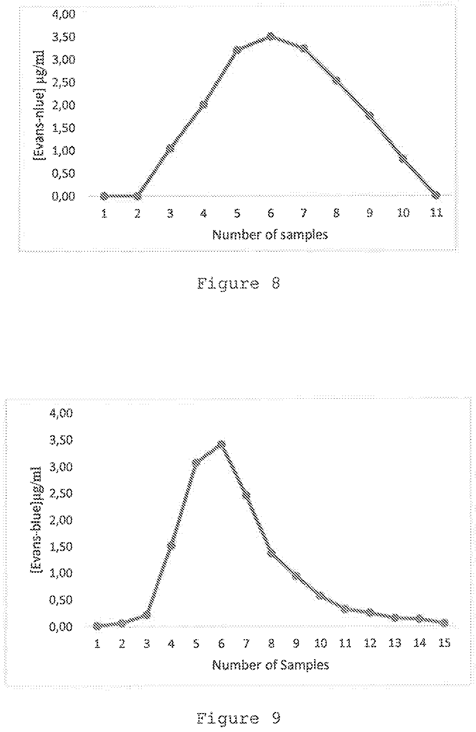

**Figure 8:** dye concentration of samples collected at 10 seconds intervals by the elution of 11 fractions of 1,0 ml of distilled water. The experience started with the elution of 1,0 ml of Evan-blue aqueous solution at concentration of 1,4 mg/100 ml by permeating a granulometry gradient of combined composite on ascendant layers (1,35 g of granulometry ($G$) > 1,0 mm$^3$; 1,80 g of 1,0 mm$^3$ > $G$ > 710 $\mu$m$^3$ ; central layer of 2,70g 710 $\mu$m$^3$ > $G$ > 500 $\mu$m$^3$ ; 0,97 g of 1,0 mm$^3$ > $G$ > 710 $\mu$m$^3$). The combined composite fulfilled a 12 cm$^3$ reactor of 4 cm$_{lengh}$ $\times$ 3 cm$_{width}$ $\times$ 1 cm$_{heigh}$ dimensions.

**Figure 9:** dye concentration of samples collected at 90 seconds intervals by the elution of 15 fractions of 1,0 ml of distilled water. The experience started with the elution of 1,0 ml of Evan-blue aqueous solution at concentration of 1,4 mg/100 ml by permeating a granuiornetry gradient of combined composite on ascendant layers (1,35 g of granulometry ($G$) > 1,0 mm$^3$; 1,80 g of 1,0 mm$^3$ > $G$ > 710 $\mu$m$^3$ ; central layer of 2,70g 500 $\mu$m$^3$ > $G$ > 300 $\mu$m$^3$ ; 1, 90 g of 1,0 mm$^3$ > $G$ > 710 $\mu$m$^3$). The combined composite fulfilled a 12 cm$^3$ reactor of 4 cm$_{lengh}$ $\times$3 cm$_{width}$ $\times$1 cm$_{height}$ dimensions.

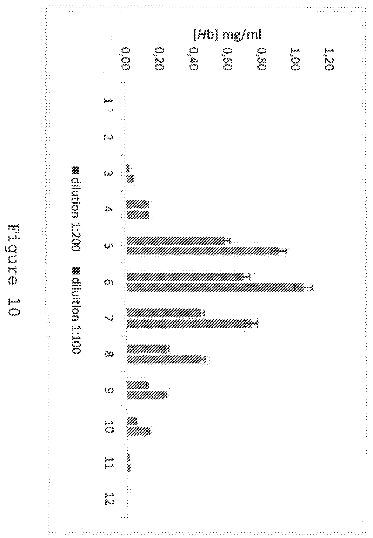

**Figure 10:** haemoglobin concentration in fractions of 1,0 ml of distilled water collected after permeation of 1,0 ml human blood at dilution of 1:100 and 1:200 (diluted in phosphate buffer saline 10 mM pH=7,3) through a granulometry gradient of combined composite on ascendant layers of 1,35 g of granulometry ($G$) > 1,0 mm$^3$; 1,80 g of 1,0 mm$^3$ > $G$ > 710 $\mu$m$^3$ ; central layer 2,70g of 710 $\mu$m$^3$ > $G$ > 500 $\mu$m$^3$ ; 0, 97 g of 1,0 mm$^3$ > G > 710 $\mu$m$^3$. The combined composite fulfilled a 12 cm$^3$ reactor of 4 cm$_{lengh}$ $\times$3 cm$_{width}$ $\times$1 cm$_{height}$ dimensions.

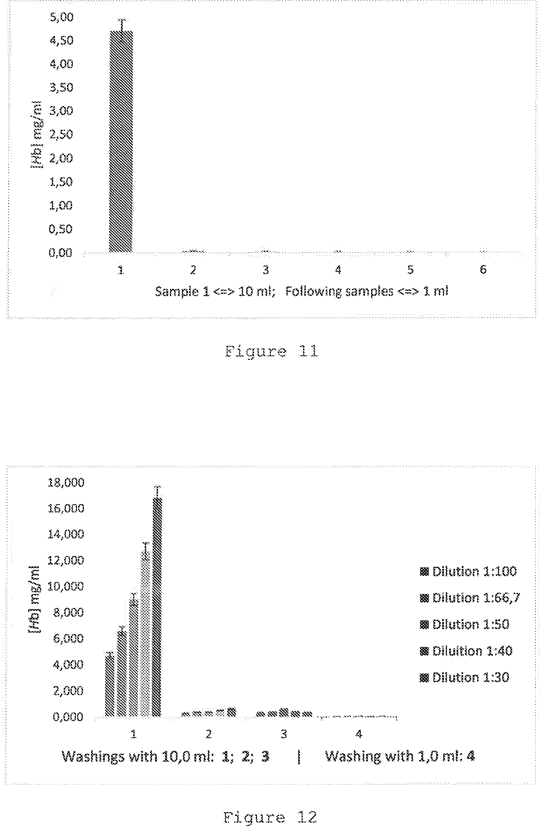

**Figure 11:** haemoglobin concentration in an initial fractions of 10,0 ml and sequent fractions of 1,0 ml of distilled water collected after permeation of 1,0 ml human blood at dilution of 1:100 (diluted in phosphate buffer saline 10 mM pH=7,3) through a granulometry gradient of combined composite on ascendant layers of 1,35 g of granulometry ($G$) > 1, 0 mm$^3$; 1,80 g of 1,0 mm$^3$ > $G$ > 710 $\mu$m$^3$ ; central layer of 2,70g 710 $\mu$m$^3$ > $G$ > 500 $\mu$m$^3$ ; 0,97 g of 1,0 mm$^3$ > G > 710 $\mu$m$^3$. The combined composite fulfilled a 12 cm$^3$ reactor of 4 cm$_{lengh}$ $\times$3 cm$_{width}$ $\times$1 cm$_{height}$ dimensions.

**Figure 12:** haemoglobin concentration in three fractions of 10,0 ml and one last fraction fractions of 1,0 ml of distilled water collected after permeation of 1,0 ml human blood at different dilutions (diluted in phosphate buffer saline 1.0 mM pH=7,3) through a granulometry gradient of combined composite on ascendant layers of 1,35 g of granulometry ($G$) > 1,0 mm$^3$; 1,80 g of 1,0 mm$^3$ > $G$ > 710 $\mu$m$^3$ ; central layer of 2,70g 710 $\mu$m$^3$ > $G$ > 500 $\mu$m$^3$ ; 0, 97 g of 1,0 mm$^3$ > $G$ > 710 $\mu$m$^3$. The combined composite fulfilled a 12 cm$^3$ reactor of 4 cm$_{lengh}$ $\times$3 cm$_{width}$ $\times$1 cm$_{height}$ dimensions.

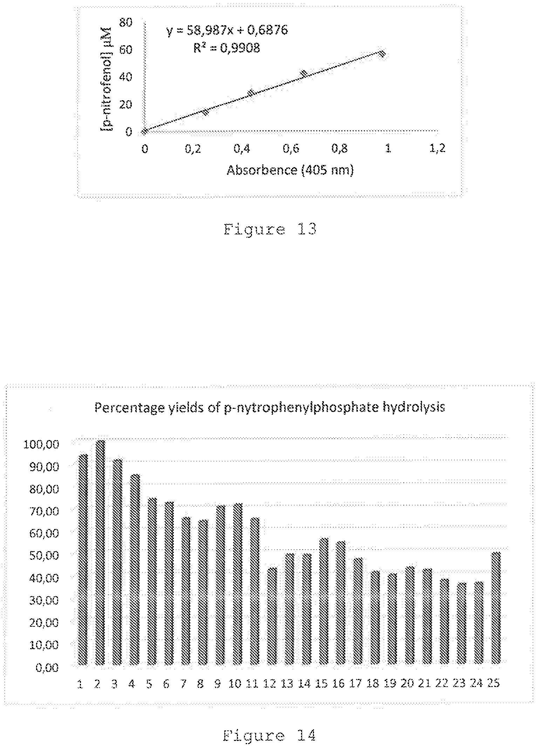

**Figure 13:** example of calibration of concentrations of the alkaline phosphatase reaction product, *p*-nitrophenol.

**Figure 14:** reaction yields of hydrolysis of *p*-nitrcphenylphosphate at concentration of 0,75 mM (in phosphate buffer 100 mM pH=9,1) added in fractions of 4,0 ml that permeated a granulometry gradient of combined composite doped with alkaline phosphatase at concentration of 0,43% (m/m). The gradient was posed in ascendant layers of 1,35 g of granulometry ($G$) > 1,0 mm$^3$; 1,80 g of 1,0 mm$^3$ > $G$ > 710 $\mu$m$^3$ ; central layer 2,70g of 500 $\mu$m$^3$ > $G$ > 300 $\mu$m$^3$ ; 1,90 g of 1,0 mm$^3$ > $G$ > 710 $\mu$m$^3$. The combined composite fulfilled a 12 cm$^3$ reactor of 4 cm$_{lengh}$ $\times$3 cm$_{width}$ $\times$1 cm$_{height}$ dimensions,

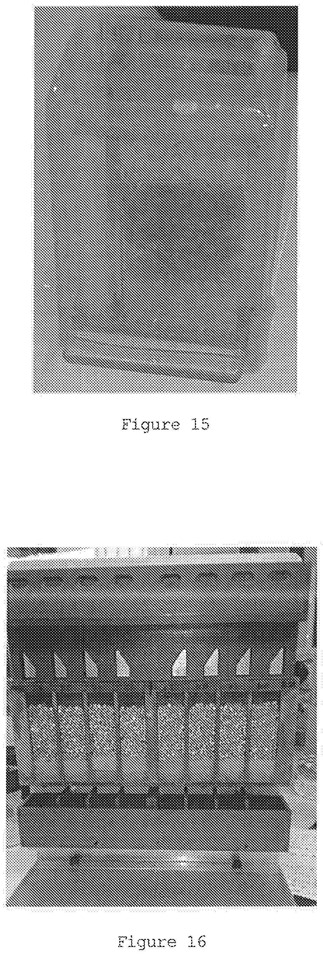

**Figure 15:** photograph of two chambers fulfilled with combined composite. The chamber on the left was filled with combined composite doped with albumin serum bovine and the chamber on the right was filled with combined composite doped with mucin. The image was obtained after an essay with supernatant of hybridoma-cells culture producing antibodies-antimucin. Ligation of secondary antibodies was revealed by chromogenic peroxidase substrate 3,3',5,5'-tetramethylbenzidine.

**Figure 16:** photograph of the device prototype in which all the chamber are posed in parallel in a disposable construct, located underneath a common liquid-collector and above the bottom-collection-tray.

## 5. Applied Examples of the Invention

### Example 1 - Composite Formulations

### Example 1.1. - Simple Composite

In the preparation of an indicative volume of 20 ml of precursors of simple composite, it was followed the protocol described by Alstein and co-workers [16] using as orthosilicate precursor 5,0 ml of tetramethyl orthosilicate (TMOS); 4,84 ml of HCl 2,5 mM; 1,0 ml of polyethyleneglycol 0,4 KDa. The mixture was agitated in a vortex until liquid medium presented transparent. Homogenization was exothermic and the mixture was then sonicated for 30 minutes. It was previously solubilized 180 to 500 mg of lyophilized protein, (e.g. enzyme, antigens/antibodies) in 10mml HEPES buffer 50 mM pH=7,5.

It was added 10 ml of the sonicated mixture to the same volume of HEPES buffer solubilizing protein. This mixture was homogenized while gelification occurred for about 6 minutes. Solidified gel was then finely fragmented and spread onto a glass surface forming a thin layer for room exposure drying. At maturation there was a loss of about 84% of volumic mass: from the initial liquid volume of 20,0 ml of precursor it was approximately obtained 3,0 g of matured composite.

The simple composite was then grinded for a granulometry lesser than 106 $\mu$m$^3$. The water content of the matured simple composite (a$_w$) was around 11 ppm. The matured simple composite had a protein concentration (mass of protein / mass of composite) among 0,5% and 1,8%.

For immune-essays it was also synthetized simple composite for negative control immobilizing bovine serum albumin with exactly the same mass of antigens/antibodies as described above.

## Example 1.2. - Combined Composite

### 1.2.1. Failures of immobilizing a composite

In tempt of synthetizing a combined composite it was followed the conventional protocol of sol-gel preparation trying to immobilize one simple composite previously synthetized instead of a free protein. Having in knowledge the proton availability required for chemical attack of the orthosilicate structure it was experimented a fraction of 50 $\mu$l of HCl 100 mM at pH=7,0 buffered media with different volumes of water for hydrolysis of 1,5 ml TMOS in order to obtain a hydrophilic composite compatible with the afore describe simple composite.

It was found a water deficit in the reacting medium proportional to the amount of mass of simple composite despite having linearly followed the initial proportion mass-of-simple-composite *versus* volume-of-precursors. Consequently there was a complete failure in TMOS hydrolysis. Table 1 presents a compilation of the experiences which elucidate the difficulties of immobilizing a simple composite in a second sol-gel.

**Table 1:** experiences in tempting to immobilize a simple composite in sol-gel.

| Volume ($\mu$l) HCl 100 mM | Volume ($\mu$l) H$_2$O$_{distilled}$ | Volume (ml) TMOS | Volume (ml) of the mixture H$_2$O/HCl + TMOS | Volume (ml) of phosphate-buffer (PB) 100 mM; pH=7,0 | Time (min) of reaction H$_2$O/HCl <-> TMOS | Mass (mg) of simple composite | RESULTS |
|---|---|---|---|---|---|---|---|
| 50 | not added | 1,5 | not controlled | not added | not controlled | not added | did not gelify |
| 50 (added to PB) | not added | 1,5 | 2,0 | 1,0 | not controlled | not added | did not gelify |
| 50 (added to H$_2$O) | 100 to 600 | 1,5 | 1,7 to 2,2 | 1,0 | not controlled | not added | variable |
| 50 (+ H$_2$O) | 540 | 1,5 | 1,0 | 1,0 | not controlled | not added | gelified |
| 50 (+ H$_2$O) | 540 | 1,5 | 1,0 | 1,0 | not controlled | 100 | gelification few consistent |
| (+ H$_2$O) | 540 | 1,5 | 1,0 | 1,0 | 7 | 100 | gelification but few robustness after drying |
| 50 (+H$_2$O) | 540 | 1,5 | 1,5 | 1,0 a 2,0 | in 2; in 3...in 7 | 100 | variable |
| 50 (+ H$_2$O) | 540 | 1,5 | 1,5 | 1,5 | 3 | 100 | gelification in drying and after |
| 625 (+ H$_2$O) | 6750 | 18,75 | 25,0 | 25,0 | 3 | 1660 | did not gelify |

(continued)

| Volume ($\mu$l) HCl 100 mM | Volume ($\mu$l) $H_2O_{distilled}$ | Volume (ml) TMOS | Volume (ml) of the mixture $H_2O$/HCl + TMOS | Volume (ml) of phosphate-buffer (PB) 100 mM; pH=7,0 | Time (min) of reaction $H_2O$/HCl <-> TMOS | Mass (mg) of simple composite | RESULTS |
|---|---|---|---|---|---|---|---|
| 63 (+ $H_2O$) | 675 | 1,87 | 2,5 | 2,5 | 3 | 166 | gelification in 1 min. Brittle composite after drying |
| 50 (+ $H_2O$) | 540 | 1,5 | 1,5 | 1,5 | 3 | 75 | gelification in 14 sec. and robustness after drying |

### 1.2.2. Practiced Formulation of the Combined Composite

The synthesis was effected with concentration of simple composite since 0,83% until 3,34% (m/v). In reference to a unit precursors volume of 3,0 ml it was dosed 25 mg to 100 mg of simple composite at granulometry lesser than 106 $\mu m^3$ and with water content ($a_w$) of circa 11 ppm. That simple composite was suspended in 1,5 ml of phosphate buffer (PB) that was prepared with 38 mM of $Na_2HPO_4.2H_2O$; 62 mM of $KH_2PO_4$. PB was further adjusted pH = 6,8 $\pm$ 0,2 at 25 °C temperature (adding 1,0 M NaOH aliquots).

It was prepared an HCl solution whose concentration depended on higrophilia of the simple composite and conditioned by respective immobilized protein. For instance but not limited, for alkaline phosphatase [HCl] = 4,5 mM ; for mucin [HCl] = 8,3 mM.

It was mixed 540 $\mu$l of distilled water with 50 $\mu$l of HCl solution and then added to 1,5 ml of TMOS. The mixture was homogenized during the initial 120 seconds and after 3 minutes 1,5 ml of that mixture was transferred to the same volume of PB suspension of simple composite. Gelification occurred in 10 to 15 seconds during which the media was homogenized in order to assure a homogeneous distribution of simple composite granules.

Polymerization went for 30 to 90 minutes after which the solidified gel was fragmented at granules of approximate volumetric dimensions less than 2 $mm^3$ and between 2 $mm^3$ and 4 $mm^3$.

At immobilization of antigens/antibodies the solidified gel was incubated in 400 rpm orbital agitation during 24 hours in bovine serum albumin (BSA) at 2,0% (m/v). The solvent was phosphate buffer saline 10 mM pH=7,3 $\pm$ 0,3 prepared with 7,6 mM $Na_2HPO_4.2H_2O$; 2,4 mM $KH_2PO_4$; 137 mM NaCl; 2,7 mM KCl. The incubating volume was in a proportion of three volumes of the solution to one volume of solidified gel and after incubation it was transferred onto a clean-dry glass surface. From there on glass surfaces were changed for three times: at 24 hours, 48h and 72h of maturation.

The maturation of the combined composites either or not incubated in BSA, ran for seven days by exposure to room conditions after which there was a loss of volumetric mass of about 70% being the final water content $a_w \approx 19$ ppm.

The protein concentration of the combined composite matured after BSA incubation was among 3,0 to 3,5 times the concentration found at the simple composite and without BSA incubation was in the range of 0,2 to 0,4.

After maturation the granule-size ($G$) separation was made and grouped in five classes:

$$G_I \Leftrightarrow (G) > 1,0 \ mm^3;$$

$$G_{II} \Leftrightarrow 1,0 \ mm^3 > G > 710 \ \mu m^3$$

$$G_{III} \Leftrightarrow G > 710 \ \mu m^3 > G > 500 \ \mu m^3$$

$$G_{IV} \Leftrightarrow G > 500 \ \mu m^3 > G > 300 \ \mu m^3$$

$$Gv \Leftrightarrow G > 300 \ \mu m^3 > G > 106 \ \mu m^3$$

## Example 2 - Time of Maturation

It was studied the loss of mass of the composites, simple and combined, along the exposure to room conditions. That monitoring was made from the moment of gelification until the assessed mass values variated less than 2,0%.

For both composites, samples of similar masses and with two orders-of-magnitude of mass were monitored in order to verify the maturation dependence from the volume of precursors (initial mass): they were studied samples of 1,5 and 3,0 ml. Figures 2 and 3 illustrate the collected data of simple and combined composites respectively.

From the obtained results it was inferred 72 and 50 hours the times of stabilization of mass-loss respectively for simple and combined composites. Consequently it was established 7 days as maturation interval for both formulation at the end of which the water contents were:

1. Simple composite $a_w$ = 11 $\pm$ 0,2 ppm;

2. Combined composite $a_w$ = 19 $\pm$ 0,6 ppm.

## Example 3 - Leaching Studies

### 3.1. Protein quantification method used to assess protein concentration of composites and supernatants

Determination of protein concentration was made by modified Lowry method [17]. However the initial procedures did not guaranty a zero-mass-balance among initially existent protein in the composite samples and the protein transferred to the supernatant resulting from thermal-alkaline digestion (composite-sample submerged in 1,0 M NaOH during 10 min at 100 °C followed by ice incubation).

Methodology used herein was optimized in order to assure that all composite-samples were digested and inherent protein content was transferred to NaOH solution: mass of composite samples not bigger than 5,0 mg with granulometry lesser than 106 $\mu m^3$. From each digestion medium 200 $\mu l$ fractions were taken to be analysed.

Calibration of protein concentrations was made with BSA standard-solution (100% purity) at concentrations from 20 to 200 $\mu g$/ml: from each concentration 200 $\mu l$. fractions were used to calibrate. Lowry-reagent was added (1,0 ml) to analysis-supernatant and BSA-standard solutions, and after 40 minutes 200 $\mu l$ of Folin-Ciocalteau reagent (diluted 1:4) was added. After 10 min. of incubation liquid samples were absorbance read at 750 nm wavelength having samples been diluted when recorded values were higher than 1,0.

### 3.2. Protein Loss by Leaching

### 3.2.1. Simple Composite

It was firstly tested the leaching with samples of simple composite immobilising three sorts of protein; bovine serum albumin (BSA), alkaline phosphatase (ALP) and generic antibodies (IgG).

The protein loads herein defined, were masses of lyophilized reagents added relatively to total volume of precursors still in sol-gel preparation. It was intended to obtain two groups of composites with different protein order-of-magnitudes of concentrations (1,0% and 10,0%.) of BSA and ALP and one third typology with immobilized antibodies (IgG) . So composite losses of protein were monitored in triple-essays in reference to different protein concentrations and using different proteins types.

The quantifications of protein contents retained in the composites were made before leaching (matured and dried composites) and after leaching essays at which the respective tested samples were 24h dried at 40 °C followed by 10 days room exposure.

Having in mind protein leaching process would be proportional to area/volume ratio of grains two granulometries were tested: above 1 mm$^3$ and under 750 $\mu m^3$.

At first instance mass samples of 30 mg were used and experiences were performed by incubating matured fragmented composites in distilled water under orbital agitation of 200 rpm during 72 hours.

The incubation liquid volume was 1,0 ml in vials of 10 ml and at the end of each essay, the liquid media was decanted and centrifuged for 11 K rpm during 10 min. From the clarified supernatants three fraction of 200 $\mu l$ were analysed in three separated quantification episodes to determine protein concentration in supernatants.

The procedures to determine after-leaching protein still retained in the composites followed the above described protocol of 5,0 ml sample-mass to ensure complete digestion of sol-gel and thus extensive release of immobilized, protein to the analytical supernatant. Again three fraction of 200 $\mu l$ were analysed in three separated protein concentration quantification episodes.

From the attained data mass-balance was made to quantify the losses of protein relative to the initial concentrations accordingly determined. On that view differentials were calculated among protein concentrations of the composites before and after leaching experiences. Additionally it was compared the amount of existent protein in the total mass of composite-samples (using previously mentioned data) before leaching and the total amount of protein in the volume of 1,0 ml incubating medium. Recorded data were averaged and respective numbers are presented at table 2.

Table 2: protein concentrations obtained at leaching tests of simple composite doped with different types of proteins and different protein concentrations.

| SIMPLE COMPOSITE | | BSA | BSA | ALP | ALP | IgG |
|---|---|---|---|---|---|---|
| [protein] in precursors (m/v) | | 1,0% | 10,0% | 1,0% | 10,0% | 0,5% |
| [protein] immobilized in composite before leaching ($\mu$g/mg) | | 14,14 | 51,08 | 13,05 | 43,11 | 9,73 |
| $\Delta$[protein] immobilized ($\mu$g/mg) BEFORE leaching – AFTER leaching | Grain > 1mm³ | 0,85 | 9,63 | 0,69 | 5,37 | 1,14 |
| | Grain < 750$\mu$m³ | 4,25 | 11,88 | 2,47 | 9,94 | 0,93 |
| $\Delta$ mass-protein ($\mu$g) Immobiliz. before leaching – supernatant | Grain > 1mm³ | 182,77 | 783,37 | 26,95 | 584,48 | 43,93 |
| | Grain < 750$\mu$m³ | 94,21 | 767,39 | 9,61 | 563,35 | 40,64 |

Those results confirmed a somehow erosion of retained protein being much relevant as smaller the grain size: concentrations recorded at composites after leaching are as closer to the initial loads as bigger the essayed grain-size.

On the other hand with samples of lower granulometry there was a smaller difference between initial loads of composites and mass of protein solubilized in supernatant which is symptomatic of more transference of protein to the leaching media. The conjunct of results for the different protein types retained in simple composite and respective different loads confirm the leaching phenomena is proportional not only to the initial protein loads but to the grain-size as well.

The recorded data regarding IgG confirm results mentioned by Alstein and co-workers [16] of an elevated retention of antibodies in this composite in which the difference before and after leaching is at the same order-of-magnitude for both granulometries.

Additionally it was tested the leaching of 5 mg and 60 mg mass-samples of simple composite doped with ALP at the maximum protein loads and minor granulometry at the same experimental conditions. The obtained numbers are presented at table 3 in which they are comparable with 30 mg mass-samples.

Table 3: protein concentrations obtained in leaching test of simple composite doped with alkaline phosphatase at the same concentration; but with samples of different mass.

| SIMPLE COMPOSITE: ALP-10% | | 5 mg | 30 mg | 60 mg |
|---|---|---|---|---|
| [protein] immobilized in composite before leaching. ($\mu$g/mg) | | 43,11 | 43,11 | 43,11 |
| BEFORE leaching - AFTER leaching | Grain < 750$\mu$m³ | 11,70 | 9,94 | 4,23 |
| | | | | |

(continued)

| SIMPLE COMPOSITE: ALP-10% | | | | |
|---|---|---|---|---|
| | | 5 mg | 30 mg | 60 mg |
| Δ mass-protein (μg) Immobilized before leaching - supernatant | Grain < 750μm³ | 28,60 | 563,35 | 1186,11 |

The view of these figures permits to conclude the loss of retained protein was influenced by the spread-of-grain in the liquid media: for the same batch volume the differences of protein concentrations retained in the composite before and after leaching are as higher as the lower mass of the essayed samples,

On the other hand with greater-mass samples it is apparent a wider difference among initial protein loads of the composites and protein concentrations at leaching supernatants that means a lesser transference of protein into the incubation media comparing to the smaller-mass samples.

The effect of minor protein erosion with greater mass of simple composite for the same reaction-volume permits to deduce the more the simple composite will be used in the same batch-reactor volume the less protein total-loss will occur and consequently more stable will be the bioprocess activity of the doped simple composite.

### 3.2.2. Combined Composite

Having in mind the comparison with quantified protein loss recorded in simple composite the same experimental procedures were followed with combined composite immobilizing simple composite doped with the same protein which would further permit to monitor also the enzyme-activity response: alkaline-phosphatase.

It was synthetized combined composite immobilizing simple composite with the highest protein load (43,11 μg/mg) to obtain two groups of samples with different protein concentrations: 8,81 μg/mg and 23,14 μg/mg. Fragmented samples of 30 mg were essayed at small grain-size similar to previous experiments. Data treatment and experimental procedures were the same as those followed with simple composite leaching monitoring and table 4 presents the obtained results.

Table 4: protein concentrations obtained in leaching test of combined composite prepared from different masses of simple composite doped with alkaline phosphatase.

| COMBINED COMPOSITE | | | |
|---|---|---|---|
| [protein]immobilized in composite before leaching (μg/mg) | | 8,81 | 23,14 |
| Δ [protein]immobilizaed (μg/mg) BEFORE leaching — AFTER leaching | Grain < 750μm³ | 0,55 | 0,78 |
| Δ mass-protein (μg) Immobilized before leaching — supernatant | Grain < 750μm³ | 201,12 | 517,21 |

The combined composite with the initial highest protein concentration presented differential values before and after leaching of circa **ten** times lower than the simple composite (0,78 μg/mg *versus* 9,94 μg/mg) having a protein concentration about **twice** lower than the simple composite (23,14 μg/mg *versus* 43,11 μg/mg).

Likewise leaching essays using 5 mg and 60 mg mass-samples of combined composite were performed with low granulometry. Table 5 presents the obtained results.

**Table 5:** protein concentrations obtained in leaching test of combined composite doped with alkaline phosphatase at concentration of 23,14 µg/mg but with samples of different mass.

| COMBINED COMPOSITE | | 5 mg | 30 mg | 60 mg |
|---|---|---|---|---|
| [proten] immobilized in composite before leaching (µg/mg) | | 23,14 | 23,14 | 23,14 |
| Δ [protein] immobilized (µg/mg) BEFORE leaching – AFTER leaching | Grain < 750µm³ | 1,31 | 0,78 | 0,12 |
| Δ mass-protein (µg) Immobilized before leaching – supernatant | Grain < 750µm³ | 43,33 | 517,21 | 1463,23 |

The view of the figures confirm that trace-loss of protein is inversely proportional to the quantity of combined composite in the same liquid volume,

The last obtained results compared with simple composite results turn out to be evident the smaller loss of protein in combined composite: differences at protein retained in the composite before and after leaching. At the same time wider differences were recorded at combined composite comparing initial immobilized protein and protein content in supernatants which reflects greater retention grades.

### 3.2.3. Exposure to an Over-concentrated Saline Medium

Simple and combined composite samples with the highest protein concentrations and smaller granulometries were exposed to high saline concentration solution 2,0 M NaCl in order to assess the loss of protein at a high ionic stress medium.

For both composites 60 mg mass-samples were tested by submerging 72 hours at 200 rpm orbital agitation, and protein quantification of leached composites and respective supernatants followed the same previous protocols. Table 6 presents the obtained data.

Table 6: protein concentrations obtained in leaching test of simple and combined composites doped with alkaline phosphatase and submerged in a solution of 2,0 M NaCl.

| Exposition to NaCl 2,0 M (samples of 60 mg) | | Simple Composite | Combined Composite |
|---|---|---|---|
| [protein]immobilized in composite before leaching (μg/mg) | | 43,11 | 23,14 |
| Δ [protein]immobilized (μg/mg) BEFORE leaching – AFTER leaching | Grain < 750μm³ | 4,90 | 0,61 |
| Δ mass-protein (μg) immobilized before leaching – supernatant | Grain < 750μm³ | 784,66 | 1018,21 |

The recorded values regarding the difference between retained protein concentrations in composites before and after exposure to saline solution are in the same order-of-magnatude of those recorded by exposure to distilled water.

Comparing numbers of mass-protein within initial composites samples and mass-protein at supernatants at the end of the experiments a smaller difference is apparent at this case which could be symptomatic of a more intense transference of protein to the incubation media as reflex of the high ionic concentration.

These figures must however be seen as leaching solution was at an over-elevated saline concentration comparative to predicted usable physiological samples and time of exposure was considerable longer than it will be used at the preferable embodiment.

**Example 4 - Catalytic Essays**

Having previously been verified that the invented formulation of combined composite has the capacity to immobilize/retain proteins, it was then intended to verify if one protein being an enzyme, is still active. In that context the activity of alkaline phosphatase (ALP; Sigma Aldrich Cat. N° 10 567 752 001) was monitored firstly as free and then as immobilized enzyme within simple and combine composites.

Enzymatic essays recurred to spectrophotometric method to quantify the concentration of $p$-nitrophenol ($p$NP) as product of the standard-reaction of $p$-nitrophenylphosphate ($p$NPP) hydrolysis having in knowledge the respective 1:1 stoichiometry.

**4.1. Enzyme immobilized in the simple composite**

After conventional free enzyme essays two sample of simple composite immobilizing ALP at precursors concentrations (m/v) of 8% and 10% were synthetized. At the end of 13 maturation days concentrations were respectively 4,8 and 6,2 mg$_{protein}$/g$_{composite}$.

Having in mind to reduce external access limitation of substrates molecules, the immobilizing matured composite samples were fragmented to grain-size at the range of 1 to 2 mm³ .The essays were performed with simple composite samples of mass from 5,0 to 8,3 g and enzyme concentrations in batch-volumes were deduced from protein concentration of respective composites and correspondent mass of the samples.

Enzymatic essays were performed in 10 ml useful-volume reactors to which 3,6 ml of same buffer solution used in free-enzyme trials (phosphate buffer 100 mM pH=9,1), was added at room temperature. Kinetic studies started at the moment 0,4 ml of substrate solution ($p$NPP, solubilized at pH=9,1 buffer) was added, and reactions ran for 28 minutes being collected 200 $\mu l$ of liquid medium at 1:30 min. intervals. Initial substrate concentrations at the total reaction volumes were identical and around 3,0 mM. Data treatment had in account that along 28 min. time the reaction medium volume was progressively reduced by the collected samples but the mass of catalytic, composite was the same.

The recorded values made possible to graph the kinetic profiles in function of enzyme loads as illustrated in Figure 4.

The obtained results turnedevident that product concentrations were proportional to the mass of composite (with immobilized enzyme) for one same initial substrate concentration.

### 4.2. Combined composite immobilising simple composite doped with enzyme

It was studied combined composite from the same formulation used at leaching tests (protein concentration of 23,14 $\mu$g/ml) and samples of mass from 14 to 20 no, were tested.

Grain-size of the samples, enzymatic essays experimental procedures and data treatment were exactly the same as those followed at simple composite kinetic studies. Similarly the quantification of existent enzyme in reaction media was made from the previously determined protein concentration of combined composite and the mass of samples used for each essay. Obtained resulted are presented in Figure 5.

Comparing maximum production of $p$NP, product of $p$NPP hydrolysis catalysed by the same enzyme immobilized in the two composite formulation it is evident that for identical masses of enzyme (11,65 mg$_{simple-composite}$; 10,8 mg$_{simple-composite}$ *versus* 12 mg$_{combined-composite}$; 11 mg$_{combined-composite}$) and at a similar time reaction (15 min. ) it was produced 8 to 9 times more of $p$NP with simple composite (40,57 mM; 26,76 mM) than with combined composite (5,03 mM; 3,03 mM).

Having in mind that combined composite of this invention is a coating of an enzyme-doped simple composite (encapsulated in a grain-size of compromise with robustness and external access of substrates) it is comprehensive that enzyme active-sites are less available and consequently, there is a lower production of enzymatic metabolite. However the lower catalytic efficiency is compensated by a more sustained retention of immobilized protein as early demonstrated by leaching studies.

### Example 5 - Drainage at Different Granulometry Profiles

It will now be presented the hydraulic tests performed with an aqueous dye solution and diluted blood permeating grained combined composite, in order to obtain residence-times compatible with utilization in a medical diagnostic device.

The combined composite herein tested was used as a filling-bed of a column also named analysis-chamber or reactor accordingly used at immune-essays or enzymatic tests. It was a rectangular box with dimensions of 4 cm$_{length}$ $\times$ 3 cm$_{width}$ $\times$ 1 cm$_{depth}$ made of transparent acrylic material that allowed to visualize the interior. The handling of its content was made by a drilled removable top-cap that once set at the box enabled elution of liquids through its interior. The surface of the opposite top was drilled as well to allow the exit of the liquids and the holes of both top-caps were 1, 0 mm diameter.

Having In reference the useful mass value of 9 grams assessed by weighting the complete filling of water of the column (volumic-mass of 1g/ml) it was planned to fill the column with grained composite. Such amount of mass was over-dimensioned relative to the application on diagnostic-device apparatus being estimated a final scale-down of 3:1. The aim of those studies was to test the drainage regime of liquid samples and thus grain-size was though as unique conditioning variable and accordingly it was used combined composite with same maturation grade.

Granulometry gradient profile was initially programmed to completely fill the useful volume and respective values were:

- Bottom layer: 15% (1,35g) $\Leftrightarrow$ grain ($G$) > 1,0 mm$^3$;

- Bottom intermedium, layer: 20% (1,80g) $\Leftrightarrow$ 1,0 mm$^3$ > $G$ > 710 $\mu$m$^3$;

- Central layer: 3,0% (2,70g) $\Leftrightarrow$ variable granulometry under 710 $\mu$m$^3$ ;

- Top intermedium layer: 20% (1,80g) $\Leftrightarrow$ 1,0 mm$^3$ > $G$ > 710 $\mu$m$^3$ ;

- Top layer: 15% (1,35g) $\Leftrightarrow$ grain > 1,0 mm$^3$.

### 5.1. Monitoring drainage of dye solution

After grain-size separation the two bottom layers were posed accordingly and the central layer was composed of grain-size ranged from 710 $\mu$m$^3$ to 212 $\mu$m$^3$. Immediate above layer was tried to be placed as programmed but only 1,6 g was able to be posed. The filling process was complied with washing with distilled water after posing each layer to improve grain compaction and reduce preferential run-off ways. Column was then left exposed 24h to 40 °C dry-heat.

The essay started by measuring the volume of water saturating the column-content (adding water to the dry grained composite content until first drops came up at the bottom) *useful liquid volume* (ULV) : 3, 0 ml was measured. The propose was to assess the volume of retained water at such gradient profile. After saturation it was evident that at each added millilitre corresponded one millilitre drained at the bottom of the column, which proved that intending to incubate the whole content of the column, it should be added a liquid volume equal to ULV.

Additionally it was monitored the run-off time elapsed by each millilitre added until at least 900 $\mu l$ were collected and such measuring was made for 25 trials at which 3 minutes was the average recorded times. Column was then left 24h exposed to 40 °C dry-heat. Composite grain-gradient was again water saturated and one 1,0 ml fraction of Evans-blue (1,4 mg/100 ml) was eluted.

Absorbance calibration ($\lambda$=608 nm) of Evans-blue solution was made (since 1:1 = 1,426 $\pm$ 6%; until 1:10 = 0,134 $\pm$ 10%) . Drainage flow of dye-solution was monitored by spectrophotometric. readings of successive eluted/collected fractions of 1, 0 ml distilled water and respective recorded values are presented in Figure 6.

After removing wet grained composite new dry grained composite was placed in the column following the same procedures except that central layer was composed by grain ranged from 7"L0 $\mu m^3$ to 300 $\mu m^3$ and the upper layer was once again exclusively composed by grain 1, 0 mm$^3$ > $G$ > 710 $\mu m^3$ but this time took 1, 16 g of grained composite. It was monitored the run-off time elapsed by each millilitre added until at least 900 $\mu l$ was collected and such measuring was made for 30 trials at which 25 seconds was the average recorded time.

Column was then left 24h exposed to 40 °C dry-heat. It was further assessed the volume of retained liquid (1,9 ml) and replicated the essay of dye-solution drainage. Collected data are presented at Figure 7.

After removing wet grained composite new dry grained composite was placed in the column following the same procedures except that central layer was composed by grain ranged from 710 $\mu m^3$ to 500 $\mu m^3$ and the upper layer was once again exclusively composed by grained composite 1, 0 mm$^3$ > $G$ > 710 $\mu m^3$ but this time took 0,98 g. It was monitored the run-off time elapsed by each millilitre added until at least 900 $\mu l$ was collected and such monitoring was made for 25 trials at which 10 seconds was the average recorded time. Column was then left 24h exposed to 40 °C dry-heat. It was further assessed the volume of retained liquid (1,0 ml) and replicated the essay of dye-solution drainage. Collected data are presented at Figure 8.

After removing wet grained composite new dry grained composite was placed in the column following the same procedures except that central layer was composed by grain ranged from 500 $\mu m^3$ to 300 $\mu m^3$ and the upper layer was once again exclusively composed by, grained composite 1,0 mm$^3$ > $G$ > 710 $\mu m^3$ but this time took 1, 90 g. It was monitored the run-off time elapsed by each millilitre added until at least 900 $\mu l$ was collected and such monitoring was made for 25 trials at which 1:30 minutes was the average recorded time.

Column was then left 24h exposed to 40 °C dry-heat. It was further assessed the volume of retained liquid (2,0 ml) and replicated the essay of dye-solution drainage. Collected data are presented at Figure 9.

All of these results indicate that for one same volume occupied by this composite and permeated by water, the amount of retained liquid and the run-off times were conditioned by the smallest grain-size layer.

Table 7 illustrate these instances where values of granulometry and respective masses of bottom and bottom-intermedium layers were maintained. At same time variating granulometry of central layers but keeping its mass-content, resulted in variation of residence-time of permeating liquid. **Table 7:** water drainage values with different grain-sizes of 2,7g central-layer of combined composite, which also affected the useful volume of retained liquid and total mass held in the column.

| Intervals of granulometry of the central layer | Total mass (g) | Volume of retained liquid (ml) | Residence time (seconds) |
|---|---|---|---|
| 710 $\mu m^3$ > $G$ > 212 $\mu m^3$ | 7,45 | 3,0 | 180 |
| 710 $\mu m^3$ > $G$ > 300 $\mu m^3$ | 7,01 | 1,9 | 30 |
| 710 $\mu m^3$ > $G$ > 500 $\mu m^3$ | 6,82 | 1,0 | 10 |
| 500 $\mu m^3$ > $G$ > 300 $\mu m^3$ | 7,75 | 2,0 | 90 |

The drainage times of 1,0 ml of dye-solution for identical granulometry intervals at the central layer:

1. in the range of about 200 $\mu m^3$ : 90 seconds (500 $\mu m^3$ > G > 300 $\mu m^3$) that compares with 10 seconds (710 $\mu m^3$ > G > 500 $\mu m^3$) ;

2. for a wider range: 180 seconds (710 $\mu m^3$ > G > 212 $\mu m^3$) that compares with 30 seconds (710 $\mu m^3$ > G > 300 $\mu m^3$).

The drainage volumes for an identical granulometry intervals:

a. in a range of about 200 $\mu m^3$ : 15 ml (500 $\mu m^3$ > G > 300 $\mu m^3$) that compares with 12 ml (710 $\mu m^3$ > G > 500 $\mu m^3$) ;

b. for a wider range: 13 ml (710 $\mu m^3$ > G > 212 $\mu m^3$) that compares with 12 ml (710 $\mu m^3$ > G > 300 $\mu m^3$) .

In summary in a compacted layer of combined composite of grain-size under 710 $\mu m^3$ the permeation volume of an aqueous sample is proportional to the magnitude of the interval of grain-sizes and inversely proportional to the respective drainage times.

It was also evident that composite total mass within the column was inversely proportional to grain-size. Such finding would be expectable in face of compaction (as bigger as the smaller the grain) and consequently less inter-particles space that enables higher densities.

**5.2. Monitoring the drainage of blood**

Based on granulometry gradient found as better flowing regime of dye-solution (central layer 710 $\mu m^3$ > G > 500 $\mu m^3$) it was tested drainage of diluted blood. Samples collected from the inventor were anticoagulated with EDTA (50 mg/ml) and diluted with phosphate buffer saline 10 mM pH=7,3. Diluted samples of 1, 0 ml were eluted followed by fractions of 1,0 ml of distilled water and run-offs were assessed by reading collected fraction at absorbance of 540 nm wavelength which targets haemoglobin (Hb) molecule. Calibration was made for Hb concentrations from 0, 6 mg/ml (absorbance = 0,314 $\pm$ 7%) to 3,0 mg/ml (absorbance = 1,635 $\pm$ 4%). Sampled blood Hb concentration was 150 mg/ml ($\pm$ 1,0%). Results recorded from three trial are presented at Figure 10.

Before these results it is evident that diluted blood run-off occurred mainly after 4,0 ml of permeating water which agrees with previous data of dye-solution drainage. So it can be concluded that permeation of blood samples at dilutions herein referred was identical to run-offs recorded with Evans-blue solution concentrated at 1,4 mg/100ml and those are two rheological identical liquid media.

Thus it seems reasonable to infer that drainage of 1:200 and 1:100 blood samples will have the same drainage regime at others gradient-grain of combined composite which enable to preview a scenario of good access to immobilized proteins (antigens/antibodies) by biological-sample fluids with viscosity and density similar to water.

**5.2.1. Monitoring the washing drainage of blood**

According to the recorded run-off profiles either with Evans-blue or with blood solutions it was studied the washing drainage of the combined composite gradient-granulometries firstly permeated by blood and then using distilled water in a first fraction of 10,0 ml followed by five sequent elutions of 1,0 ml. At the series of three essays performed with 1:100 diluted blood it was collected 96 $\pm$ 0,5% of added Hb right at the first fraction of 10,0 ml as illustrated in Figure 11.

Based on those results it was then studied the washing of the grained composite using lesser diluted samples of blood down to 1:30. The protocol in between every experimental episode, the grained content of the column was abundantly washed with distilled water and then 35 °C dried for 24 hours. Previous to addition, of a new blood-sample the grained composite was saturated with 20,0 ml of distilled water.

It was monitored the clearance of the collected fractions at elution of three fractions of 10,0 ml of distilled water and one last of 1,0 ml and the obtained results are presented at Figure 12 .

From the collected data it is evident that even for the most concentrated blood-samples the drainage of 1,0 ml analytical blood-unit-volume occurred mostly with elution of 10,0 ml water being that in third and fourth collected fractions, the Hb concentrations were lower than 1,0 mg/ml.

Having in mind these tests were performed with a total column-mass-filling of 6,82 g and the scale of these tests was also approximately 3:1 then at the final utilization scale the estimated mass of combined composite for the same gradient (relative proportions) will range among 1,5 g to 3,0 g. At this view and in a linear scaling of granulometry gradients herein studied., it is reasonable to estimate an effective washing volume of 10 ml.

**Example 6 - Combined Composite in Enzymatic Reactor**

At this example it was studied the enzymatic activity for at least 20 cycles of activity of the combined composite filling afore described acrylic column and supplied by fractions of substrate-solution that permeated the grained-composite pushed by atmospheric pressure in a vertical plug-flow regime. The propose was to quantify the reaction-yield of conversion of p-nitrophenylphosphate (pNPP) into p-nitrophenol (pNP) catalysed by alkaline phosphatase immobilized in combined composite.

It was used the enzyme concentration threshold of correspondence among specific activity and load-of-enzyme:

0,4% (m/m) [18] [19]. It was used the grain-size gradient previously found as the best run-off for enzymatic process: residence time of at least 1,5 minutes (central layers granulometry 500 $\mu m^3$ > G > 300 $\mu m^3$). Total amount of combined composite mass was 7,75 g and its compacting was effected to minimize preferential run-off ways. The volume of retained liquid was 2,0 ml.

After filling the column the experience started with abundant wash of the grained gradient composite at room temperature with the same eluent previously used in similar catalytic experience (phosphate buffer 100 mm pH = 9,1) and herein used as eluent too. It was then added 4,0 ml of substrate solution (solubilized in pH = 9,1) and after 15 minutes incubation the grained composite was permeated by three fractions of 2,0 ml of eluent followed by eight fractions of 1,0 ml. Quantification of metabolite concentration was direct from absorbance readings once the enzymatic reaction product was collected at one same volume added at respective elution fraction (contrarily to the batch process early described) .

Data treatment started by calibration of concentrations of the product (pNP) for absorbance values ($\lambda$=405 nm) under 1,0: up to 56 $\mu$M (see the example Figure 13).

$Abs_{405\,nm}$ values attained in each of the 11 collected fractions were converted to pNP concentration. Having in known the volume of each collected fraction it was computed the number of moles existent in each collected fraction. It was summed the number of collected moles of reaction product, Additionally knowing the substrate (pNPP) concentration it was computed the respective number of moles initially supplied based of the solution-volume fed to the reactor (see table 8).

Table 8: data treatment of the first experiment results at bioreactor of combined composite immobilizing alkaline phosphatase, on hydrolysis of p-nitrophenylphosphate.

| [ALP] (mg/g) | 4,31 | Mass of pNPP (mg) | 5,9 |
| | | Molar-weight pNPP (g/mol) | 371,14 |
| | | Solvent volume (mL) | 50 |
| | | [pNPP] (M) | 0,0003179 |
| | | Volume of added solution (mL) | 4 |
| | | Number of pNPP added moles | 1,272E-06 |

| Slope | Origin ordinate | [pNPP] (μM) | 0,318 |
| --- | --- | --- | --- |
| 58,58 | 0,0016 | | |

| Collections | | | [pNP] | Number of |
| N° | Dilution | $Abs_{405\,nm}$ | M | moles pNP |
| --- | --- | --- | --- | --- |
| 1 (2 mL) | 1 | 0,0748 | 5,10E-06 | 1,01997E-08 |
| 2 (2 mL) | 1 | 0,0829 | 5,58E-06 | 1,11552E-08 |
| 3 (2 mL) | 1 | 0,0756 | 5,15E-06 | 1,0294E-08 |
| 4 | 1 | 0,0723 | 4,95E-06 | 9,90472E-09 |
| 5 | 1 | 0,0708 | 4,86E-06 | 9,72776E-09 |
| 6 | 1 | 0,0631 | 4,41E-06 | 8,81936E-09 |
| 7 | 1 | 0,0575 | 4,08E-06 | 8,15871E-09 |
| 8 | 1 | 0,0498 | 3,63E-06 | 7,25031E-09 |
| 9 | 1 | 0,0495 | 3,61E-06 | 7,21491E-09 |
| 10 | 1 | 0,0389 | 2,98E-06 | 5,96439E-09 |
| 11 | 1 | 0,0314 | 2,54E-06 | 5,07958E-09 |
| | | | 9,37687E-08 | TOTAL |

Knowing this reaction is 1:1 stoichiometry percentage conversion yields were calculated: product, number of moles * 100 / substrate number of moles. At the first trial it was used a substrate concentration of 318 $\mu$M and the recorded yield was 7,4 %. Next trial used a substrate solution one order-of-magnitude higher: 3,03 mM and the yield was 19,5 %.

The third trial kept substrate concentration and prolonged incubation period for 20 minutes. The recorded yield was 25,7 %. From that result it was inferred longer incubation time allowed a more extensive hydrolysis of the added substrate. Fourth trial was a replication of the third and the recorded yield was 24,1 %.

At the fifth trial substrate concentration was reduced to ½ and the recorded yield was 23,1%, and at the next trial with the same conditions the value recorded was 29,7%.

At seventh trial substrate concentration was reduced to ¼ : 750 $\mu$M. The recorded yield was 36,4 %. That last trail was replicated for twice and recorded yields were: 38,9% and 35,6%.

At one next experimental episode three trial were performed replicating the last protocol and recorded yields were: 32,9% ; 28,8% and 28,2%. At one new series of five trials the recorded yields were: 25,4% ; 24,9%; 27,5%; 27,8% and 25,3%. At one other experimental episode of three trial the recorded yields were: 16,6% ; 19,0% and 18,9%,

At the two following trails the recorded yields were: 21,7% and 21,1%. At one last experimental episode nine trial were performed, and the recorded yields were: 18,2% ; 16,0% ; 15,5% ; 16,7% ; 16,3% ; 14,6% ; 13,9% ; 14,0% and 19,0%.

Compilation of those mentioned figures is presented at Figure 14 for the normalized procedures established after the seventh trial in which it was used a 750 $\mu$M substrate concentration and 20 minutes incubation time. The graphic shows recorded yields in reference to the value obtained at second experiment of that series (38,9%) that holds as maximum value: 100%.

Before these results it is reasonable to conclude that combined composite is consistently applicable, at immobilization of an enzyme, alkaline phosphatase, while retaining its activity. Having additionally in mind that experimental normalization was attained after seven essays of procedure adjustments it becomes reasonable to preview the utilization or this composite formulation for a wider number of cycles of more than those 20 initially pointed.

**Example 7 - Preferred Utilisation of Combined Composite at Diagnostic Device**

Now it will be described one preferential utilization of the invented combined composite applied to medical diagnosis onto a portable device constructed as a conjunct of vertical parallel operating units. It complies the maximum of 4 units and each unit includes two chambers, one analytical and one negative control chamber.

All the chamber are filled with grained combined composite in which the analytical one is filled with combined composite immobilizing an immunologically responsive protein, an antigen or an antibody associated with the diagnosis of a human being or superior animal pathology and the negative-control chamber is filled with combined composite immobilizing an immunologically non-responsive protein.

The functioning of the device is based on immunediagnosis principles where a biological sample suspected to carry antibodies (or antigens) related to a pathology, by operating the device those molecules will ligate to respective specific ligands. The latter will be antigens (or antibodies) immobilized in the combined composites whose physical characteristics of pore-size enable the permeation of those liquid biological samples. After antigen-antibody ligation a primary immune-complex will be formed and the composite must be washed to remove the excess of debris and non-ligated proteins.

At the first instance of this example it was preliminary tested the response of the combined composite immobilizing a simple composite doped with mucin [20] 1, 8% (m/m). The combined composite was used to fulfil a test-column homologous to one device-unit. The test-column was filled with grained composite with granulometry gradient-profile analogous to blood drainage tests.

The immobilized antigen mucin is amolecule with a glycidic structure of sialic-acids homologous to surface-ligands of tumour cells (e.g. breast cancer). It was tested the formation of primary complex by ligation of an antibody-reagent kindly supplied by Glycoiirununo logy-Group of Science and Technology Faculty of Universidade Nova de Lisboa. Such antibodies had previously demonstrated a high ligation affinity to neoplasia tissues [21]. The biologic samples at those tests were the supernatants from the culture of antibody-producer animal cells (hybridoma-cells).

After incubation of biological sample, the grained composite was washed to remove the excess of non-ligated antibodies, The detection of the formed immune-complexes was made by addition of a secondary antibody labelled with peroxidase. Such antibody had specific affinity to the referred primary antibody. The addition of the secondary antibody provided the formation of a secondary immune-complex: antigen immobilized - antibody *biological-sample* - antibody *labelled*. The grained composite was washed once again to remove the excess of non-ligated proteins,

It was then added the chromogenic substrate of peroxidase: 3,3',5,5'-tetramethyl-benzidine. The substrate was degraded by the secondary antibody and consequently conferred blue-green colouring to the combined composite thus revealing the existence of the primary antibodies in the biological sample. The procedure was validated by the similar test performed with homologous grained combined composite doped with the non-immunogenic protein bovine serum albumin, at which the combined composite did not gain any colour as illustrated by Figure 15.

Recycling the grained combined composite enabled the respective re-utilization and that procedure was made by elution of a chaotropic solution that disrupted the ligations of primary and secondary antibodies. Grained combined composites were then washed to remove all the released proteins.

### 7.1. Preliminary Essay Protocol

At the preferential utilization of the invented combined composite in medical diagnosis preliminary essays must be performed in order to test Sensitivity and Specificity criteria,

Samples, of combined composite immobilizing one same simple composite with a unique concentration of antigen / antibody / BSA will be tested. Protocol includes 8 essay-vials having each vial a sample of 100 mg of grained composite with granulometry range of 100 $\mu$m$^3$ to 300 $\mu$m$^3$:

i) four essay-vials in which one of each vial will have a sample of grained combined composite immobilizing respectively 25; 50; 75; 100 mg of simple composite with BSA. Those masses of simple composite are relative to 3,0 ml of precursors when combined composite was synthetized.

ii) four essay-vials in which one of each vial will have a sample of grained combined composite immobilizing respectively 25; 50; 75; 100 mg of simple composite with antigen / antibody. Those masses of simple composite are relative to 3,0 ml of precursors when combined composite will be synthetized.

### 7.1.1. Reaction solutions

Phosphate Buffer Saline 10 mM pH=7,3 $\pm$ 0,3 (25 °C) + 0,05% (m/v) Tween-20 (PBS-T) used at:

- Dilution of the biological samples;

- Dilution of the secondary labelled antibody;

- Washing the grained combined composite.

Chromogenic peroxidase substrate: 3,3',5,5'-tetramethy benzidine (TMB) used under dilution with distilled water.

Recycling solution Restore™ *Western Blot Stripping Buffer*; Thermo Scientific (Sb) used under dilution with distilled water.

### 7.1.2. Experimental procedures by vial

Step 1: Conditioning of the composite.
Grained combined composite will be humidified with 2$\times$ 2,5 ml PBS-T: Incubation time: 5 rain. Liquid medium will then be decanted.

Step 2: Elution of the biological sample.
Biological liquid sample will initially be diluted (in PBS-T) at 1:10 for the respective volume of 2,5 ml. It is intended to obtain a result of unequivocal colouring of the composite doped with antigen (or antibody) and unequivocal clearance of the composite doped with BSA and so dilution grade of biological samples will be optimized since 1:5 to 1:13. Incubation time: 20 min. Liquid medium will then be decanted.

Step 3: First wash with 2$\times$ 2,5 ml PBS-T. Liquid medium will then be decanted,

Step 4: Elution of the secondary antibody.
Secondary antibody labelled with peroxidase will initially be diluted (in PBS-T) at 1:3333 for the respective volume of 2,5 ml. It is intended to obtain a result of unequivocal colouring of the composite doped with antigen (or antibody) and unequivocal clearance of the composite doped with BSA and so dilution grade will be optimized since 1:2000 to 1:5000. Incubation time: 5 ruin. Liquid medium will then be decanted.

Step 5: Second wash with 4× 2,5 ml PBS-T. Liquid medium will then be decanted.

Step 6: Elution of the chromogenic substrate.
Peroxidase chromogenic substrate, will initially be diluted with distilled water at 1:4 for the respective volume of 2,5 ml. It is intended to obtain a result of unequivocal colouring of the composite doped with antigen (or antibody) and unequivocal clearance of the composite doped with BSA and so TMB dilution grade will be optimized to 1:3. Incubation time: 10 to 20 min. Liquid medium will then be decanted.

Results: colouring of the combined composite is consequence of immune-complexes formation. Intensity of the attained colour in each of the essayed composites samples will give indication of analytical criteria of the method:

**Sensitivity** - the lesser mass of simple composite imprisoned at the combined composite (trendily 25 mg) that will enable to detect the existence of antibodies (or antigens) in biological samples;

**Specificity** - the greatest mass amount of simple composite imprisoned at the combined composite (trendily 100 mg) that will reveal no cross-reaction at:

I. no colouring of the negative-control combined composite;
II. no colouring of combined composite tested with biological samples containing different specificity antibodies (or antigens).

Step 7: Recycling.

Striping buffer, will initially be diluted with distilled water at 1:16 for the respective volume of 2,5 ml. According to the obtained clearance Sb dilution will be optimized since 1:10 to 1:32. Incubation time: 10 min. Liquid medium will then be decanted.

Step 8: Third wash with 6× 2,5 ml PBS-T. Liquid medium will then be decanted.

**7.1.3. Filling of chambers**

This example refers the operation of the diagnosis medical device conceived for a maximum capacity of eight chamber as early mentioned. Each chamber had the useful internal volume of 9 cm$^3$ and was filled as forwardly described.
After seven days maturation the grained combined composite was washed with distilled water and then dried for 3 hours at 37 °C. Grain-size separation defined 5 granulometry classes:

$$G_I \Leftrightarrow G > 1,0 \text{ mm}^3;$$

$$G_{II} \Leftrightarrow 1,0 \text{ mm}^3 > G > 710 \text{ µm}^3$$

$$G_{III} \Leftrightarrow G > 710 \text{ µm}^3 > G > 500 \text{ µm}^3$$

$$G_{IV} \Leftrightarrow G > 500 \text{ µm}^3 > G > 300 \text{ µm}^3$$

$$G_V \Leftrightarrow G > 300 \text{ µm}^3 > G > 106 \text{ µm}^3$$

The filling of each chamber was composed by 6 layers as referred below from top to bottom with respective mass of grain-size:

- Layer 6: $G_I \Leftrightarrow$ 200 mg;
- Layer 5: $G_{III} \Leftrightarrow$ 450 mg;
- Layer 4: $G_{IV} \Leftrightarrow$ 450 mg;
- Layer 3: $G_V \Leftrightarrow$ 120 mg;

- Layer 2: $G_{II} \Leftrightarrow$ 210 mg;
- Layer 1: $G_{I} \Leftrightarrow$ 350 mg;

The compaction of each layer was optimized by eluting 3,0 to 5,0 ml of distilled water reducing so the formation of preferential run-off ways. Once deposited all the layers rest free-volume of the chamber was filled (with glass spheres of 0,8 to 1,2 mm diameter) up to 1,0 cm from the top. That upper space was left free in order to have a visible regurgitation window.

The whole piece of 8 chamber was left 48 hours at 37 °C and after that retained liquid volumes were quantified by eluting 10 ml of distilled water. By measuring after the collected water the differentials were 2,0 $\pm$ 0, 4 ml.

### 7.1.4. Operation of the device

Biological samples placed at liquid-collector drained directly to analytical-chambers posed underneath as presented in Figure 16 photograph. The drainage of liquids along all the experimental procedures did not fulfil the regurgitation windows in order avoid lateral contamination between chambers. The forwardly described experimental procedures refer the operation of one chamber and the dilution grades were previously determined in preliminary essays.

The minimal liquid volumes utilized on operation of the device were twice the values indicated below as each analysis-chamber was operated simultaneously with respective negative-control-chamber.

Step 1: Conditioning of the composite.
Grained combined composites were humidified with 10,0 ml PBS-T.

Step 2: Elution of the biological sample.
Biological liquid samples were diluted (in PBS-T) at 1:5 up to 1:13 for the respective volume of 4,0 ml. Incubation time: 20 min.

Step 3: First wash with 10,0 ml PBS-T.

Step 4: Elution of the secondary antibody.
Mother solution of secondary antibody labelled with peroxidase was DBS-T diluted at 1:2000 up to 1:5000 for the respective volume of 4,0 ml. Incubation time: 5 min.

Step 5: Second wash with 2 fractions of 10,0 ml PBS-T.

Step 6: Elution of the chromogenic substrate.
Peroxidase chromogenic substrate was diluted with distilled water at 1:4 up to 1:3 for the respective volume of 4,0 ml. Incubation time: 10 to 20 min.

Results: analytical-chambers filled with grained combined composites doped with mucin gained blue-green colour approximately proportional to mucin concentration and analytical-chambers filled with grained combined composites doped with BSA at mucin-concentration correspondence gained no colour.

Step 7: Recycling.
Striping buffer was diluted with distilled water at 1:16 up to 1:10 for the respective volume of 7,0 ml. Incubation time: 10 min.

Step 8: Third wash with 3 fractions of 10,0 ml PBS-T.

For the operation of the whole 8 chambers the liquid-volumes used were at the order of magnitude of linear correlation to the values referred to one chamber.

### 8. References

[1] Rassy, H., Perrard, A., Pierre, A. 2003. Behaviour of Silica Aerogel Network as Highly Porous Solid Solvent Media for Lipases in a Model Transesterification Reaction. Chem. Biochem. 44: 203 - 210.

[2] Reetz, M., Zonta, A., Simplekamp, J., Konen, W. 1996. In situ fixation of lipase-containing hydrophobic sol-gel materials on stirred glass-highly efficient heterogeneous biocatalyst. Chem. Comm. 11: 1397 - 1398.

[3] Reetz, M., Tielmann, P., Wiesenhöfer, W., Könen, W., Zonta, A. 2003. Second Generation Sol-Gel Encapsulated Lipases: Robust Heterogeneous Biocatalysts. Adv. Synth. Catal. 345, 717 - 728.

[4] Bergogne, L., Fennouh, S., Guyon, S., Roux, C., Livage, J. 2001. Sol-gel entrapment of enzymes. Materials Research Society Symposium Proceedings 628: (Organic/Inorganic Hybrid Materials) CC10.2.1 - CC10.2.6.

[5] T.M. Butler, B.D. MacCraith, C. McDonagh, J. Non-Cryst. Solids 224 (1998) 249.

[6] Lobnik, I, Oehme, I. Murkovic, O.S. Wolfbeis, Anal. Chim. Acta 367 (1998) 159.

[7] G.E. Badini, K.T.V. Grattan, A.C.C. Tseung, Analyst 120 (1995) 1025.

[8] N. Aharonson, M. Altstein, G. Avidan, D. Avnir, A. Bronshtein, A. Lewis, K. Lieberman, M. Ottolenghi, Y. Polevaya, C. Rottman, J. Samuel, S. Shalom, A. Strinkovski, A. Turiansky, Mater. Res. Soc. Symp. Proc. 346 (1994) 519.

[9] M.M. Collinson, Mikrochim. Acta 129 (1998) 149.

[10] U. Schubert, N. Hüsing, A. Lorenz, Chem. Mater. 7 (1995) 2010.

[11] M. Plaschke, R. Czolk, J. Reichert, H.J. Ache. Thin Solid Films 279 (1996) 233.

[12] P.J. Skrdla, S.S. Saavedra, N.R. Armstrong. Appl. Spectrosc. 53 (1999) 785.

[13] Muditha D. Senarath-Yapa, S. Scott Saavedra. Analytica Chimica Acta 432 (2001) 89-94.

[14] T. Nguyen, K.P. McNamara, Z. Rosenzweig. Anal. Chim. Acta 400 (1999) 45.

[15] Johannes, T., Simurdiak, M., Zhao, H. Encyclopedia of Chemical Processing DOI: 10.1081/E-ECHE-120017565Copyright. (2006)101-102.

[16] Alstein, M., Bronshtein, A., Glattshein, B., Zeichner, A., Tamiri, T., Almog, J. Immunochemical Approaches for Purification and Detection of TNT traces by Antibodies Entrapped in a Sol-Gel Matrix. Anal. Chem. (2001) 73, 2461-2467.

[17] Lowry, O.H., Rosenbrough, N.J., Farr, A.L., Randall. R.J. Protein measurement with the Foullin phenol reagent. J. Biol., Chem. (1951)193: 265-275.

[18] Reetz, M., Zonta, A., Simpelkamp, J. Biotechnol. Bioeng. (1996) 49, 527-534.

[19] Barreira, G., Ferreira, A. Vidinha, P., Cabral, J., Martinho, J., Lima, J., Cabrita, E., Barreiros, S. Acessing Diffusion in Enzyme Sol-gel Matrices. RSC Advances (2014) 25099-25105.

[20] Mucin, Bovine Submaxillary Gland, Calbiochem® Cat: 4999643-500MG.

[21] Antibody produced by Glicoimunology Group, of Faculty of Science and Technology of Unlversidade NOVA de Lisboa and submitted to Portuguese patent request N.° 110526, "ANTIBODY, FUNCTIONAL FRAGMENT OR PROBE THEREOF AGAINST TUMOUR ANTIGENS",

**Claims**

1. A method of production of a combined composite for stabilization of active or activable biological materials comprising the steps of:

    a) providing a simple composite in the form of a sol-gel immobilizing uniquely by encapsulation active or activable biological materials of one or more different natures, finely divided and with low water content;

b) providing a second sol-gel immobilizing the simple composite of a), comprising:

i) preparing a suspension of simple composite of a) in a phosphate-buffer solution 100 mM pH = 6,8 ± 0,2 at a mass/volume concentration between 0,83% and 3,34% in reference to the volume of the final reacting mixture;

ii) effecting the hydrolysis of tetramethyl orthosilicate by acidic catalysis adding to tetramethyl orthosilicate an HCl solution at a concentration of 4,50mM to 8,34 mM in the proportion of 28% of the final volume of this mixture;

iii) adding the mixture from ii) to an equal volume of the suspension from i) at 25°C to 30°C allowing polymerization to occur until the obtained combined composite has a consistency that enables it to be fragmented;

iv) fragmenting the combined composite obtained from iii) to granules of two classes of size: equal and smaller than approximately 2 mm$^3$ and approximately between 2 mm$^3$ and 4 mm$^3$;

v) optionally, incubating the fragmented combined composite of iv) during 24 hours in bovine serum albumin (BSA) 2,0% m/v solubilized in saline phosphate buffer 10 mM pH=7,3 in a proportion of three volumes of the incubating solution to one volume of combined composite;

vi) maturating the fragmented combined composite by drying on a surface until a mass-decrease of about 70% and available water content ($a_w$) of about 20 ppm;

whereby a combined composite is obtained doubly immobilizing in sol-gel uniquely by encapsulation said active or activable biological materials, wherein the loss by leaching of said biological materials is reduced, being preserved its activity or activation capability.

2. The method according to claim 1 wherein in said combined composite the loss by leaching of said doubly immobilized biological materials is reduced and its activity or capability of activation is preserved for at least 20 cycles of use.

3. The method according to claim 1 or 2 wherein the simple composite of a) has an available water content ($a_w$) of 10 to 15 ppm.

4. The method according to any of the previous claims wherein the simple composite of a) has a granulometry of from 100 to 110 $\mu$m$^3$.

5. The method according to any of the previous claims wherein in step b) iii) the polymerization occurs in about 30 to 90 minutes.

6. The method according to any of the previous claims wherein the active or activable biological material is one or more from a biologically active protein such as an enzyme, a co-enzyme, and a immunologically active protein such as an antigen, an antibody, a hapten-protein, or any other member of a specific biological relationship.

7. The method according to claim 6 wherein the active or activable biological material is one or more from a immunologically active protein such as an antigen, an antibody, a hapten-protein and the method includes step v).

8. A combined composite that doubly immobilizes in sol-gel and uniquely by encapsulation active or activable biological material, obtainable by a method of any of claims 1 to 7.

9. The combined composite that doubly immobilizes in sol-gel and uniquely by encapsulation active or activable biological material **characterized in that** the loss by leaching of said immobilized biological material is reduced and its activity is preserved for at least 20 cycles of use.

10. The combined composite that doubly immobilizes in sol-gel and uniquely by encapsulation active or activable biological material according to any of claims 8 or 9 wherein the immobilized active or activable biological material is one or more from a biologically active protein such as an enzyme, a co-enzyme, and an immunologically active protein such as an antigen, an antibody, a hapten-protein, or any other member of a specific biological relationship.

11. The combined composite that doubly immobilizes in sol-gel and uniquely by encapsulation active or activable biological material according to any of claims 8 to 10 wherein the immobilized simple composite, matured and finely divided has a granulometry of from 100 $\mu$m$^3$ to 110 $\mu$m$^3$.

12. The combined composite that doubly immobilizes in sol-gel and uniquely by encapsulation active or activable biological material according to any of claims 8 to 11 wherein said active or activable biological material is one or more of biologically active proteins such as an enzyme or a co-enzyme and the combined composite is to be used in biocatalysis.

13. The combined composite that doubly immobilizes in sol-gel and uniquely by encapsulation active or activable biological material according to any of claims 8 to 11 wherein said active or activable biological material is a specifically binding immunologically active protein and said combined composite is to be used for detection of its respective specific ligands.

14. The combined composite that doubly immobilizes in sol-gel and uniquely by encapsulation active or activable biological material according to claim 13 wherein said specifically binding immunologically active protein is an antigen, an antibody, a hapten-protein, and said combined composite is to be used for diagnosis of a pathology.

15. The combined composite that doubly immobilizes in sol-gel and uniquely by encapsulation active or activable biological material according to any of claims 8 to 14 or produced by a method according to any of claims 1 to 7 for use in medical diagnosis.

16. A method of diagnosis of a pathology in a human or superior animal subject comprising the steps of:

a) contacting a sample of biological fluid from the subject suspected to contain an analyte related to a pathology, in particular plasma/serum of blood, urine, supernatant of tissue maceration or any other fluid obtained from the subject, with the combined composite that doubly immobilize in sol-gel biological material that specifically binds to said analyte wherein the combined composite is a composite of any of the claims 8 to 14 or produced by a method according to any of the claims 1 to 7; and
b) detecting of specific binding between the analyte present in the sample and the biological material immobilized in the combined composite, the presence or absence of said specific binding indicating, respectively, an affirmative or negative diagnosis of the pathology in the subject.

17. The method of diagnosis according to claim 16 wherein the pathology is any disease that results from the presence of antigens and/or antibodies in the fluids and/or tissues of a human or superior animal subject.

18. The method of diagnosis according to claim 16 or 17, wherein the analyte in the sample is an antigen or an antibody and an immune-complex results from the specific binding.

19. The method of diagnosis according to claim 18, wherein the detection of specific binding comprises the steps of:

a) contacting the (primary) immune-complex with a secondary antibody labelled with a detectable tracer, that binds specifically to the analyte of the biological sample; and
b) detecting the tracer in the sample, directly or via its interaction with a particular reagent.

20. The method of diagnosis according to claim 19, wherein the detectable tracer in the secondary antibody is an enzyme and its detection is effected by revelation of colour after contact with its chromogenic substrate, or is a photo-sensible molecule and its detection is effected by fluorescence emission after luminous excitation with correspondent wavelength.

21. The method of diagnosis according to any of claim 16 to 20, additionally comprising a step c) of recycling the combined composite and the replication of steps a) to c) for at least 20 times.

22. An analytical chamber for detection of specific ligands of an immunologically active protein comprising as filling a combined composite immobilizing an immunologically active protein of any of claims 8 to 14 or produced by a method according to any of claims 1 to 7, for use in medical diagnosis.

23. Device for detection of specific ligands of immunologically active proteins comprising a maximum of four groups of two chambers constructed in parallel, each of said groups being comprised of one chamber of analysis according to claim 22 and one chamber of negative control filled with combined composite immobilizing an immunologically non-active protein, for use in medical diagnosis.

**24.** The device according to claim 23 wherein each immunologically active protein immobilized in each combined composite filling each analytical chamber is associated to a different specific pathology.

**25.** The device according to claim 23 or 24 **characterized in that** it is reused for at least 20 times.

**Patentansprüche**

**1.** Verfahren zur Herstellung eines kombinierten Verbundwerkstoffs zur Stabilisierung aktiver oder aktivierbarer biologischer Materialien, das folgende Schritte umfasst:

a) Bereitstellen eines einfachen Verbundwerkstoffs in Form eines Sol-Gels, das aktive oder aktivierbare biologische Materialien einer oder mehrerer verschiedener Arten, einzigartig durch Verkapselung, immobilisiert, fein verteilt und mit niedrigem Wassergehalt;

b) Bereitstellen eines zweiten Sol-Gel, das den einfachen Verbundwerkstoff a) immobilisiert, umfassend:

i) Herstellen einer Suspension des einfachen Verbundwerkstoffs a) in einer 100 mM PhosphatPufferlösung mit pH = 6,8 ± 0,2 bei einer Masse/ Volumenkonzentration zwischen 0,83% und 3,34%, bezogen auf das Volumen der endgültigen Reaktionsmischung;

ii) Bewirken der Hydrolyse von Tetramethylorthosilikat durch saure Katalyse, wobei dem Tetramethylorthosilikat eine HCl-Lösung mit einer Konzentration von 4,50 mM bis 8,34 mM im Anteil von 28% des Endvolumens dieser Mischung zugesetzt wird;

iii) Zugabe der Mischung ii) zu einem gleichen Volumen der Suspension i) bei einer Temperatur von 25 °C bis 30 °C, wobei die Polymerisation so lange laufen soll, bis der erhaltene kombinierte Verbundwerkstoff eine Konsistenz aufweist, die seine Fragmentierung ermöglicht;

iv) Fragmentierung des aus iii) erhaltenen kombinierten Verbundwerkstoffs in Körnchen zweier Größenklassen: gleich und kleiner als etwa 2 mm3 und etwa zwischen 2 mm3 und 4 mm3;

v) gegebenenfalls Inkubation des fragmentierten kombinierten Verbundwerkstoffs iv) für 24 Stunden in Rinderserumalbumin (BSA) 2,0% m/v, gelöst in 10 mM Salzphosphatpuffer mit pH=7,3 in einem Verhältnis von drei Volumina der Inkubationslösung zu einem Volumen des kombinierten Verbundwerkstoffs;

vi) Reifung des fragmentierten kombinierten Verbundwerkstoffs durch Trocknen auf einer Oberfläche bis eine Masseverringerung von etwa 70% und ein verfügbarer Wassergehalt (aw) von etwa 20 ppm erreicht sind;

wodurch ein kombinierter Verbundwerkstoff erhalten wird, der die aktiven oder aktivierbaren biologischen Materialien durch Verkapselung in Sol-Gel zweifach immobilisiert, was den Verlust durch Auslaugung der biologischen Materialien reduziert und deren Aktivität oder Aktivierungsfähigkeit erhalten bleibt.

**2.** Verfahren nach Anspruch 1, wobei in dem genannten kombinierten Verbundwerkstoff der Verlust durch Auslaugung der doppelt immobilisierten biologischen Materialien reduziert ist und deren Aktivität oder Aktivierungsfähigkeit für mindestens 20 Anwendungszyklen erhalten bleibt.

**3.** Verfahren nach Anspruch 1 oder 2, wobei der einfache Verbundwerkstoff a) einen verfügbaren Wassergehalt (aw) von 10 bis 15 ppm aufweist.

**4.** Verfahren nach einem der vorhergehenden Ansprüche, wobei der einfache Verbundwerkstoff a) eine Korngrössenverteilung von 100 bis 110 μm3 aufweist.

**5.** Verfahren nach einem der vorhergehenden Ansprüche, wobei in Schritt b) iii) die Polymerisation in etwa 30 bis 90 Minuten erfolgt.

**6.** Verfahren nach einem der vorhergehenden Ansprüche, wobei das aktive oder aktivierbare biologische Material ein oder mehrere Teile eines biologisch aktiven Proteins darstellt, wie z. B. ein Enzym, ein Coenzym und ein immunologisch aktives Protein, wie z. B. ein Antigen, einen Antikörper, ein Hapten-Protein oder irgendein anderes Mitglied einer spezifischen biologischen Verwandtschaft.

**7.** Verfahren nach Anspruch 6, wobei das aktive oder aktivierbare biologische Material eines oder mehrere Teile eines immunologisch aktiven Protein darstellt, wie ein Antigen, ein Antikörper, ein Hapten-Protein ist, und das Verfahren den Schritt v) umfasst.

8. Ein kombinierter Verbundwerkstoff, der in Sol-Gel doppelt und einzigartig durch Verkapselung aktives oder aktivierbares biologisches Material immobilisiert, das durch ein Verfahren nach einem der Ansprüche 1 bis 7 erhältlich ist.

9. Kombinierter Verbundwerkstoff, der in Sol-Gel doppelt und einzigartig durch Verkapselung aktives oder aktivierbares biologisches Material immobilisiert, **dadurch gekennzeichnet, dass** der Verlust durch Auslaugen des genannten immobilisierten biologischen Materials reduziert ist und dessen Aktivität für mindestens 20 Verwendungszyklen erhalten bleibt.

10. Kombinierter Verbundwerkstoff, der gemäss den Ansprüchen 8 oder 9 aktives oder aktivierbares biologisches Material in Sol-Gel doppelt und einzigartig durch Verkapselung immobilisiert, wobei das aktive oder aktivierbare biologische Material ein oder mehrere Teile eines biologisch aktiven Proteins darstellt, wie z. B. ein Enzym, ein Coenzym und ein immunologisch aktives Protein, wie z. B. ein Antigen, einen Antikörper, ein Hapten-Protein oder irgendein anderes Mitglied einer spezifischen biologischen Verwandtschaft.

11. Kombinierter Verbundwerkstoff, der gemäss den Ansprüchen 8 bis 10 aktives oder aktivierbares biologisches Material in Sol-Gel doppelt und einzigartig durch Verkapselung immobilisiert, wobei der immobilisierte einfache Verbundwerkstoff, gereift und fein verteilt, eine Korngrössenverteilung von 100 $\mu$m3 bis 110 $\mu$m3 aufweist.

12. Kombinierter Verbundwerkstoff, der gemäss den Ansprüchen 8 bis 11 aktives oder aktivierbares biologisches Material in Sol-Gel doppelt und einzigartig durch Verkapselung immobilisiert, wobei das aktive oder aktivierbare biologische Material ein oder mehrere Teile eines biologisch aktiven Proteins darstellt, und der kombinierte Verbundwerkstoff in der Biokatalyse verwendet werden soll.

13. Kombinierter Verbundwerkstoff, der gemäss den Ansprüchen 8 bis 11 aktives oder aktivierbares biologisches Material in Sol-Gel doppelt und einzigartig durch Verkapselung immobilisiert, wobei das aktive oder aktivierbare biologische Material ein spezifisch bindendes immunologisches Protein ist, und der genannte Verbundwerkstoff zur Bestimmung seiner spezifischen Bindungsproteine verwendet werden soll.

14. Kombinierter Verbundwerkstoff, der gemäss Anspruch 13 aktives oder aktivierbares biologisches Material in Sol-Gel doppelt und einzigartig durch Verkapselung immobilisiert, wobei das spezifisch bindende immunologisch aktive Protein ein Antigen, ein Antikörper, ein Hapten-Protein ist, und der kombinierte Verbundwerkstoff zur Diagnose einer Pathologie verwendet werden soll.

15. Kombinierter Verbundwerkstoff, der gemäss den Ansprüchen 8 bis 14 aktives oder aktivierbares biologisches Material in Sol-Gel doppelt und einzigartig durch Verkapselung immobilisiert, oder durch ein Verfahren nach einem der Ansprüche 1 bis 7 zwecks Verwendung in der medizinischen Diagnose hergestellt wurde.

16. Verfahren zur Diagnose einer Pathologie in einem menschlichen oder höher tierischen Subjekt:

a) In-Kontakt-Bringen einer Probe einer biologischen Flüssigkeit des Subjekts, bei der Verdacht besteht, dass sie einen Analyten enthält, der mit einer Pathologie in Zusammenhang steht, insbesondere Plasma/Serum von Blut, Urin, Überstand einer Gewebemazeration oder jede andere Flüssigkeit, die vom Subjekt erhalten wird, mit dem kombinierten Verbundwerkstoff, der biologisches Material, das sich spezifisch an den Analyten bindet, in Sol-Gel doppelt immobilisiert, wobei der kombinierte Verbundwerkstoff ein Verbundwerkstoff nach einem der Ansprüche 8 bis 14 ist oder durch ein Verfahren nach einem der Ansprüche 1 bis 7 hergestellt wird; und
b) Bestimmung der spezifischen Bindung zwischen dem in der Probe vorhandenen Analyten und dem in dem kombinierten Verbundwerkstoff immobilisierten biologischen Material, wobei das Vorhandensein oder die Abwesenheit der spezifischen Bindung eine positive bzw. negative Diagnose der Pathologie im Subjekt anzeigt.

17. Diagnoseverfahren nach Anspruch 16, wobei die Pathologie eine beliebige Krankheit ist, die Folge des Vorhandensein von Antigenen und/oder Antikörpern in den Flüssigkeiten und/oder Geweben eines menschlichen oder höheren tierischen Subjekts ist.

18. Diagnoseverfahren nach Anspruch 16 oder 17, wobei der Analyt in der Probe ein Antigen oder ein Antikörper ist, und ein Immunkomplex Folge der spezifischen Bindung ist.

**19.** Diagnoseverfahren nach Anspruch 18, wobei die Bestimmung der spezifischen Bindung folgende Schritte umfasst:

a) In-Kontakt-Bringen des (primären) Immunkomplexes mit einem sekundären Antikörper, der mit einem nachweisbaren Tracer (Markierstoff) markiert ist und sich spezifisch sich an den Analyten der biologischen Probe bindet; und
b) Nachweis des Tracers in der Probe, entweder direkt oder über seine Wechselwirkung mit einem bestimmten Reagenz.

**20.** Diagnoseverfahren nach Anspruch 19, wobei der nachweisbare Tracer im sekundären Antikörper ein Enzym ist, und sein Nachweis durch Farboffenbarung nach Kontakt mit seinem chromogenen Substrat erfolgt, oder ein lichtempfindliches Molekül ist, dessen Nachweis durch Fluoreszenzemission nach Lichtanregung mit entsprechender Wellenlänge erfolgt.

**21.** Diagnoseverfahren nach einem der Ansprüche 16 bis 20, das zusätzlich einen Schritt c) zwecks Recycling des kombinierten Verbundwerkstoffs umfasst sowie die Wiederholung der Schritte a) bis c) für mindestens 20 Mal umfasst.

**22.** Analysekammer zur Bestimmung spezifischer Bindungsproteine eines immunologisch aktiven Proteins, welches als Füllung einen kombinierten Verbundwerkstoff beinhaltet, der ein immunologisch aktives Protein nach einem der Ansprüche 8 bis 14 immobilisiert, oder durch ein Verfahren nach einem der Ansprüche 1 bis 7 hergestellt wurde, zwecks Verwendung in der medizinischen Diagnose.

**23.** Vorrichtung zur Bestimmung spezifischer Bindungsproteine immunologisch aktiver Proteine, die maximal vier Gruppen von zwei parallel aufgebauten Kammern umfasst, wobei jede der Gruppen aus einer Kammer für die Analyse nach Anspruch 22 und einer Kammer für die Negativkontrolle besteht, die mit einem kombinierten Verbundwerkstoff gefüllt ist, der ein immunologisch nicht aktives Protein immobilisiert, zwecks Verwendung in der medizinischen Diagnose.

**24.** Vorrichtung nach Anspruch 23, wobei jedes immunologisch aktive Protein, das in jedem kombinierten Verbundwerkstoff, der jede Analysekammer füllt, immobilisiert ist, mit einer anderen spezifischen Pathologie assoziiert ist.

**25.** Vorrichtung nach Anspruch 23 oder 24, **dadurch gekennzeichnet, dass** sie mindestens 20 Mal wiederverwendet werden kann.

## Revendications

**1.** Procédé de production d'un composite combiné pour la stabilisation de matériels biologiques actifs ou activables, comprenant les étapes suivantes:

a) fournir un composite simple sous forme de sol-gel immobilisant uniquement par encapsulation des matériels biologiques actifs ou activables d'une ou plusieurs natures différentes, finement divisé et à faible teneur en eau;
b) fournir un second sol-gel immobilisant le composite simple de a), comprenant:

i) préparer une suspension du composite simple de a) dans une solution tampon de phosphate 100 mM pH = 6,8 $\pm$ 0,2 à une concentration masse/volume comprise entre 0,83% et 3,34% par rapport au volume du mélange réactionnel final;
ii) effectuer l'hydrolyse de l'orthosilicate de tétraméthyle par catalyse acide en ajoutant à l'orthosilicate de tétraméthyle une solution d'HCl à une concentration de 4,50 mM à 8,34 mM à raison de 28% du volume final de ce mélange;
iii) ajouter le mélange de ii) à un volume égal de la suspension de i) de 25°C à 30°C permettant la polymérisation de se produire jusqu'à ce que le composite combiné obtenu ait une consistance qui permet sa fragmentation;
iv) fragmenter le composite combiné obtenu en iii) en obtenant granulés de deux classes de taille: égale et inférieure à environ 2 mm$^3$ et environ entre 2 mm$^3$ et 4 mm$^3$;
v) éventuellement, incuber le composite combiné fragmenté de iv) pendant 24 heures dans de l'albumine de sérum bovin (BSA) à 2,0% m/v solubilisée dans du tampon salin au phosphate 10 mM pH=7,3 à raison de trois volumes de la solution d'incubation pour un volume de composite combiné;

vi) maturer le composite combiné fragmenté par séchage sur une surface jusqu'à une diminution de masse d'environ 70% et une teneur en eau disponible ($a_w$) d'environ 20 ppm;

de ce fait obtenant un composite combiné immobilisant doublement dans un sol-gel uniquement par encapsulation lesdits matériels biologiques actifs ou activables, dans lequel la perte par lixiviation desdits matériels biologiques est réduite, tout en préservant son activité ou sa capacité d'activation.

2. Procédé selon la revendication 1 dans lequel, dans ledit composite combiné, la perte par lixiviation desdits matériels biologiques doublement immobilisés est réduite et son activité ou sa capacité d'activation est préservée pendant au moins 20 cycles d'utilisation.

3. Procédé selon la revendication 1 ou 2, dans lequel le composite simple de a) présente une teneur en eau disponible ($a_w$) de 10 à 15 ppm.

4. Procédé selon l'une quelconque des revendications précédentes dans lequel le composite simple de a) présente une granulométrie de 100 à 110 $\mu m^3$.

5. Procédé selon l'une quelconque des revendications précédentes, dans lequel, à l'étape b) iii), la polymérisation se produit en environ 30 à 90 minutes.

6. Procédé selon l'une quelconque des revendications précédentes, dans lequel le matériel biologique actif ou activable est un ou plusieurs choisis parmi une protéine biologiquement active telle qu'une enzyme, une coenzyme et une protéine immunologiquement active telle qu'un antigène, un anticorps, une protéine haptène, ou tout autre élément d'une relation biologique spécifique.

7. Procédé selon la revendication 6, dans lequel le matériel biologique actif ou activable est un ou plusieurs choisis parmi une protéine immunologiquement active telle qu'un antigène, un anticorps, une protéine haptène, et le procédé comprend l'étape v).

8. Composite combiné immobilisant doublement en sol-gel et uniquement par encapsulation du matériel biologique actif ou activable, susceptible d'être obtenu par un procédé selon l'une quelconque des revendications 1 à 7.

9. Composite combiné immobilisant doublement en sol-gel et uniquement par encapsulation du matériel biologique actif ou activable **caractérisé en ce que** la perte par lixiviation dudit matériel biologique immobilisé est réduite et son activité est préservée pendant au moins 20 cycles d'utilisation.

10. Composite combiné immobilisant doublement en sol-gel et uniquement par encapsulation du matériel biologique actif ou activable selon l'une quelconque des revendications 8 ou 9, dans lequel le matériel biologique actif ou activable immobilisé est un ou plusieurs choisis parmi une protéine biologiquement active telle qu'une enzyme, une coenzyme et une protéine immunologiquement active telle qu'un antigène, un anticorps, une protéine haptène ou tout autre élément d'une relation biologique spécifique.

11. Composite combiné immobilisant doublement en sol-gel et uniquement par encapsulation du matériel biologique actif ou activable selon l'une quelconque des revendications 8 à 10, dans lequel le composite simple immobilisé, maturé et finement divisé a une granulométrie de 100 $\mu m^3$ à 110 $\mu m^3$.

12. Composite combiné immobilisant doublement en sol-gel et uniquement par encapsulation du matériel biologique actif ou activable selon l'une quelconque des revendications 8 à 11, dans lequel ledit matériel biologique actif ou activable est un ou plusieurs choisis parmi des protéines biologiquement actives telles qu'une enzyme ou une coenzyme, et le composite combiné est destiné à être utilisé dans la biocatalyse.

13. Composite combiné immobilisant doublement en sol-gel et uniquement par encapsulation du matériel biologique actif ou activable selon l'une quelconque des revendications 8 à 11, dans lequel ledit matériel biologique actif ou activable est une protéine immunologiquement active à liaison spécifique et ledit composite combiné est destiné à être utilisé pour la détection de ses ligands spécifiques respectifs.

14. Composite combiné immobilisant doublement en sol-gel et uniquement par encapsulation du matériel biologique actif ou activable selon la revendication 13, dans lequel ladite protéine immunologiquement active à liaison spécifique est un antigène, un anticorps, une protéine haptène, et ledit composite combiné est destiné à être utilisé pour le diagnostic d'une pathologie.

15. Composite combiné immobilisant doublement en sol-gel et uniquement par encapsulation du matériel biologique actif ou activable selon l'une quelconque des revendications 8 à 14, ou qui est produit par un procédé selon l'une quelconque des revendications 1 à 7, destiné à être utilisé en diagnostic médical.

16. Méthode de diagnostic d'une pathologie chez un sujet humain ou animal supérieur comprenant les étapes suivantes:

   a) mise en contact d'un échantillon de fluide biologique provenant du sujet suspecté de contenir un analyte lié à une pathologie, notamment du plasma/sérum de sang, d'urine, de surnageant de macération tissulaire ou tout autre fluide obtenu du sujet, avec un composite combiné immobilisant doublement en sol-gel du matériel biologique qui se lie spécifiquement audit analyte, le composite combiné étant un composite selon l'une quelconque des revendications 8 à 14 ou étant produit par un procédé selon l'une quelconque des revendications 1 à 7; et
   b) détection d'une liaison spécifique entre l'analyte présent dans l'échantillon et le matériel biologique immobilisé dans le composite combiné, la présence ou l'absence de ladite liaison spécifique indiquant, respectivement, un diagnostic positif ou négatif de la pathologie chez le sujet.

17. Méthode de diagnostic selon la revendication 16 dans laquelle la pathologie est toute maladie résultant de la présence d'antigènes et/ou d'anticorps dans les fluides et/ou tissus d'un sujet humain ou d'un animal supérieur.

18. Méthode de diagnostic selon la revendication 16 ou 17, dans laquelle l'analyte dans l'échantillon est un antigène ou un anticorps, et un complexe immunitaire résulte de la liaison spécifique.

19. Méthode de diagnostic selon la revendication 18, dans laquelle la détection d'une liaison spécifique comprend les étapes suivantes:

   a) mise en contact le complexe immunitaire (primaire) avec un anticorps secondaire marqué avec un traceur détectable, qui se lie spécifiquement à l'analyte de l'échantillon biologique; et
   b) détection du traceur dans l'échantillon, directement ou via son interaction avec un réactif particulier.

20. Méthode de diagnostic selon la revendication 19, dans laquelle le traceur détectable dans l'anticorps secondaire est une enzyme et sa détection est effectuée par révélation de couleur après contact avec son substrat chromogène, ou est une molécule photosensible et sa détection est effectuée par émission de fluorescence après excitation lumineuse de longueur d'onde correspondante.

21. Méthode de diagnostic selon l'une quelconque des revendications 16 à 20, comprenant en outre une étape c) de recyclage du composite combiné et la répétition des étapes a) à c) pendant au moins 20 fois.

22. Chambre d'analyse pour la détection de ligands spécifiques d'une protéine immunologiquement active, comprenant comme remplissage un composite combiné immobilisant une protéine immunologiquement active selon l'une quelconque des revendications 8 à 14, ou qui est produit par un procédé selon l'une quelconque des revendications 1 à 7, destinée à être utilisée en diagnostic médical.

23. Dispositif de détection de ligands spécifiques de protéines immunologiquement actives comprenant un maximum de quatre groupes de deux chambres construites en parallèle, chacun desdits groupes étant constitué d'une chambre d'analyse selon la revendication 22 et d'une chambre de contrôle négatif remplie d'un composite combiné immobilisant une protéine immunologiquement non active, destiné à être utilisé dans le diagnostic médical.

24. Dispositif selon la revendication 23 dans lequel chaque protéine immunologiquement active immobilisée dans chaque composite combiné remplissant chaque chambre d'analyse est associée à une pathologie spécifique différente.

25. Dispositif selon les revendications 23 ou 24 **caractérisé en ce qu'**il est réutilisé au moins 20 fois.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- PT 110526

### Non-patent literature cited in the description

- **B et al.** *Biotechnology Letters,* 2000, vol. 22 (24), 1953-1958
- **SHI J et al.** *Nanotechnology,* vol. 22 (35), 355502
- **RASSY, H. ; PERRARD, A. ; PIERRE, A.** Behaviour of Silica Aerogel Network as Highly Porous Solid Solvent Media for Lipases in a Model Transesterification Reaction. *Chem. Biochem.,* 2003, vol. 44, 203-210
- **REETZ, M. ; ZONTA, A. ; SIMPLEKAMP, J. ; KONEN, W.** In situ fixation of lipase-containing hydrophobic sol-gel materials on stirred glass-highly efficient heterogeneous biocatalyst. *Chem. Comm.,* 1996, vol. 11, 1397-1398
- **REETZ, M. ; TIELMANN, P. ; WIESENHÖFER, W. ; KÖNEN, W. ; ZONTA, A.** Second Generation Sol-Gel Encapsulated Lipases: Robust Heterogeneous Biocatalysts. *Adv. Synth. Catal.,* 2003, vol. 345, 717-728
- **BERGOGNE, L. ; FENNOUH, S. ; GUYON, S. ; ROUX, C. ; LIVAGE, J.** Sol-gel entrapment of enzymes. *Materials Research Society Symposium Proceedings,* 2001, vol. 628
- **T.M. BUTLER ; B.D. MACCRAITH ; C. MCDONAGH.** *J. Non-Cryst. Solids,* 1998, vol. 224, 249
- **LOBNIK, I ; OEHME, I. MURKOVIC ; O.S. WOLFBEIS.** *Anal. Chim. Acta,* 1998, vol. 367, 159
- **G.E. BADINI ; K.T.V. GRATTAN ; A.C.C. TSEUNG.** *Analyst,* 1995, vol. 120, 1025
- **N. AHARONSON ; M. ALTSTEIN ; G. AVIDAN ; D. AVNIR ; A. BRONSHTEIN ; A. LEWIS ; K. LIEBERMAN ; M. OTTOLENGHI ; Y. POLEVAYA ; C. ROTTMAN.** *Mater. Res. Soc. Symp. Proc.,* 1994, vol. 346, 519
- **M.M. COLLINSON.** *Mikrochim. Acta,* 1998, vol. 129, 149
- **U. SCHUBERT ; N. HÜSING ; A. LORENZ.** *Chem. Mater.,* 2010, vol. 7 (1995
- **M. PLASCHKE ; R. CZOLK ; J. REICHERT ; H.J. ACHE.** *Thin Solid Films,* 1996, vol. 279, 233
- **P.J. SKRDLA ; S.S. SAAVEDRA ; N.R. ARMSTRONG.** *Appl. Spectrosc.,* 1999, vol. 53, 785
- **MUDITHA D. SENARATH-YAPA ; S. SCOTT SAAVEDRA.** *Analytica Chimica Acta,* 2001, vol. 432, 89-94
- **T. NGUYEN ; K.P. MCNAMARA ; Z. ROSENZWEIG.** *Anal. Chim. Acta,* 1999, vol. 400, 45
- **JOHANNES, T. ; SIMURDIAK, M. ; ZHAO, H.** *Encyclopedia of Chemical Processing,* 2006, 101-102
- **ALSTEIN, M. ; BRONSHTEIN, A. ; GLATTSHEIN, B. ; ZEICHNER, A. ; TAMIRI, T. ; ALMOG, J.** Immunochemical Approaches for Purification and Detection of TNT traces by Antibodies Entrapped in a Sol-Gel Matrix. *Anal. Chem.,* 2001, vol. 73, 2461-2467
- **LOWRY, O.H. ; ROSENBROUGH, N.J. ; FARR, A.L. ; RANDALL. R.J.** Protein measurement with the Foullin phenol reagent. *J. Biol., Chem.,* 1951, vol. 193, 265-275
- **REETZ, M. ; ZONTA, A. ; SIMPELKAMP, J.** *Biotechnol. Bioeng.,* 1996, vol. 49, 527-534
- **BARREIRA, G. ; FERREIRA, A. ; VIDINHA, P. ; CABRAL, J. ; MARTINHO, J. ; LIMA, J. ; CABRITA, E. ; BARREIROS, S.** Acessing Diffusion in Enzyme Sol-gel Matrices. *RSC Advances,* 2014, 25099-25105